# EUROPÄISCHE PATENTSCHRIFT

(11) **EP 4 384 318 B1**
(45) Veröffentlichungstag und Bekanntmachung des Hinweises auf die Patenterteilung: **01.10.2025**
(21) Anmeldenummer: 22758554.4
(22) Anmeldetag: 09.08.2022
(51) Int. Cl.: B01L 3/00, B01F 33/451, C12N 15/10, G01N 1/40

(54) **ISOLIEREN VON ANALYTEN UNTERSCHIEDLICHER ANALYT-KLASSEN**
ISOLATION OF ANALYTES OF DIFFERENT ANALYTE CLASSES
ISOLEMENT D'ANALYTES DE DIFFÉRENTES CLASSES D'ANALYTES

(30) Priorität: 13.08.2021 DE 102021208893
(43) Veröffentlichungstag der Anmeldung: 19.06.2024
(73) Patentinhaber: Hahn-Schickard-Gesellschaft für angewandte Forschung e. V., 70569 Stuttgart (DE)
(72) Erfinder: PAUST, Nils, 79115 Freiburg (DE); JÜLG, Peter, 79102 Freiburg (DE); LÜDDECKE, Jan, 79395 Neuenburg am Rhein (DE); HUTZENLAUB, Tobias, 77955 Ettenheim (DE); SCHLENKER, Franziska, 79341 Kenzingen (DE); ARJMAND, Ehsan Mahmodi, 79108 Freiburg (DE); GRETHER, Gustav, 79379 Mühlheim (DE); KARLE, Marc, 79424 Auggen (DE)
(74) Vertreter: Stöckeler, Ferdinand
(86) Internationale Anmeldenummer: PCT/EP2022/072377
(87) Internationale Veröffentlichungsnummer: WO 2023/017049

(56) Entgegenhaltungen:
- US-A1- 2014 030 788
- WOO HYUN-KYUNG ET AL: "Exodisc for Rapid, Size-Selective, and Efficient Isolation and Analysis of Nanoscale Extracellular Vesicles from Biological Samples", ACS NANO, vol. 11, no. 2, 17 January 2017 (2017-01-17), US, pages 1360 - 1370, XP055948413, ISSN: 1936-0851, DOI: 10.1021/acsnano.6b06131
- WANG WENSHUO ET AL: "Recent Progress in Isolation and Detection of Extracellular Vesicles for Cancer Diagnostics", ADVANCED HEALTHCARE MATERIALS, vol. 7, no. 20, 1 October 2018 (2018-10-01), DE, pages 1800484, XP055974247, ISSN: 2192-2640, Retrieved from the Internet <URL:https://api.wiley.com/onlinelibrary/tdm/v1/articles/10.1002%2Fadhm.201800484> DOI: 10.1002/adhm.201800484
- SHU ZHU ET AL: "Microfluidics for label-free sorting of rare circulating tumor cells", ANALYST, vol. 145, no. 22, 1 January 2020 (2020-01-01), UK, pages 7103 - 7124, XP055750399, ISSN: 0003-2654, DOI: 10.1039/D0AN01148G

## Beschreibung

Die vorliegende Erfindung betrifft Verfahren und Vorrichtungen zum Isolieren unterschiedlicher Analyt-Klassen aus dem gleichen Probenvolumen, und insbesondere Verfahren und Vorrichtungen zum Isolieren von extrazellulären Vesikeln, EV, und/oder zirkulierenden Tumorzellen als eine erste Analyt-Klasse und von zellfreien Nukleinsäuren als eine zweite Analyt-Klasse aus dem gleichen Probenvolumen einer biologischen Probe.

### Einleitung

Die frühe Diagnostik von Erkrankungen ist häufig entscheidend für die Heilungschancen von Patienten, weshalb ein dringender Bedarf nach hoher diagnostischer Sensitivität besteht. Zudem ist eine spezifische Diagnostik von Erkrankungen relevant um richtige Therapieentscheidungen zu treffen, sowie falsch-positive Befunde auf ein Minimum zu begrenzen, da diese mit einer großen Belastung der Patienten und hohen Zusatzkosten für das Gesundheitssystem durch Folgeuntersuchungen einhergehen.

Es besteht somit zusätzlich zur hohen Sensitivität ein Bedarf an hoher diagnostischer Spezifität. Die Sensitivität und Spezifität eines diagnostischen Tests werden durch die Bestimmung eines gemeinsamen Grenzwertes definiert, welcher Testergebnisse in positiv und negativ unterteilt. Dadurch sind Sensitivität und Spezifität miteinander verknüpft und können nicht unabhängig voneinander optimiert werden, so lange die Diagnose auf einen einzelnen Analyten gestützt ist.

Eine vielversprechende Möglichkeit sowohl diagnostische Sensitivität als auch Spezifität anzuheben, ist die Analyse von mehreren Analyten bzw. Analyt-Klassen in einer Patientenprobe und zu einem Zeitpunkt. Dies erfordert jedoch in der Regel entweder das Aufteilen der Patientenprobe in mehrere Subvolumina oder das Sammeln größerer Probenvolumina, wodurch nicht nur Kosten entstehen, beispielsweise mehrere Blutröhrchen, sondern insbesondere die Belastung für den Patienten steigt. Dieses Problem wird dadurch verstärkt, dass bei den heute bekannten, krankheits-assoziierten Analyten aus nicht- bzw. minimal-invasiven Proben ohnehin eine sehr große und für die Patienten belastende Menge an Probe gesammelt wird, z.B. 10 mL Vollblut für die Analyse von cfDNA (zellfreie Desoxyribonukleinsäure) in Plasma. Hintergrund ist die oftmals sehr geringe Konzentration der Analyten innerhalb der Probenmatrix, insbesondere in frühen Stadien der Erkrankung bzw. im Anschluss an eine Therapie (Überwachung der Minimalen Resterkrankung, MRD = minimal residual disease).

Beispielsweise können zentrifugal-mikrofluidische Systeme eingesetzt werden, um Analyten für eine Analyse aus einer biologischen Probe bereitzustellen und/oder Analyten zu analysieren. Unter einem zentrifugal-mikrofluidischen System wird dabei ein System verstanden, das ausgelegt ist, um unter Ausnutzung der bei einer Rotation entstehenden Zentrifugalkraft Flüssigkeiten zu handhaben. Die zentrifugale Mikrofluidik beschäftigt sich mit der Handhabung von Flüssigkeiten im Picoliter- bis Milliliter-Bereich in rotierenden Systemen. Solche Systeme sind meist Polymer-Einwegkartuschen, die in oder anstelle von Zentrifugenrotoren verwendet werden, mit der Absicht, Laborprozesse zu automatisieren. Dabei können Standardlaborprozesse, wie Pipettieren, Zentrifugieren, Mischen oder Aliquotieren in einer mikrofluidischen Kartusche implementiert werden. Zu diesem Zweck beinhalten die Kartuschen Kanäle für die Fluidführung, sowie Kammern für das Auffangen von Flüssigkeiten. Allgemein können solche Strukturen, die zur Handhabung von Fluiden ausgelegt sind, als Fluidikstrukturen bezeichnet werden. Allgemein können solche Kartuschen als Fluidikmodule bezeichnet werden.

Die Kartuschen können mit einer vordefinierten Abfolge von Drehfrequenzen, dem Frequenzprotokoll, beaufschlagt werden, so dass die in den Kartuschen befindlichen Flüssigkeiten durch die Zentrifugalkraft bewegt werden können. Anwendung findet die zentrifugale Mikrofluidik hauptsächlich in der Laboranalytik und in der mobilen Diagnostik.

### Stand der Technik

Verfahren und Vorrichtungen zum Isolieren von Analyten aus biologischen Proben unter Verwendung von zentrifugal-mikrofluidischen Systemen sind bekannt.

Chi-Ju Kim et al., "Fully automated, on-site isolation of cfDNA from whole blood for cancer therapy monitoring", Lab Chip, 2018, 18, Seiten 1321 bis 1329, offenbaren ein zentrifugal-mikrofluidischen Chip und ein zugehöriges Gerät, mit welchem vollautomatisch und in weniger als 30 Minuten cfDNA aus Vollblut isoliert werden kann. Hierbei wird die cfDNA auf Silica-Beads gebunden, gewaschen und eluiert. Die Fluidik basiert auf Membranventilen, die durch einen Stößel geöffnet oder geschlossen werden.

Hyun-Kyung Woo et al., "Exodisc for Rapid, Size-Selective, and Efficient Isolation and Analysis of Nanoscale Extracellular Vesicles from Biological Sample", ACS Nano 2017, 11, Seiten 1360 bis 1370, offenbaren ein Fluidikmodul in Form einer Scheibe, die Fluidikstrukturen aufweist, um EVs, extrazelluläre Vesikel, mittels zentrifugaler Mikrofluidik zu isolieren. In dem Fluidikmodul sind zweier Nanofilter vorgesehen, mittels derer EVs im Größenbereich von 20 nm bis 600 nm aus biologischen Proben innerhalb von 30 Minuten isoliert werden. Hierbei werden zunächst große Partikel mittels eines ersten Filters aus der Probe entfernt und nachfolgend mittels eines zweiten Filters EVs isoliert. Anschließend werden die EVs gewaschen und eluiert. Die Fluidik basiert auf Membranventilen, die durch einen Stößel geöffnet oder geschlossen werden.

Es gibt ferner Verfahren, bei denen zwei oder mehr Analyt-Klassen aus einer einzelnen Probe isoliert werden.

Aus der EP 3 167 062 B1 ist ein Verfahren bekannt, bei dem Partikel, Zellen und/oder Zellstücke oder andere Verunreinigungen durch Zentrifugation oder Filtration aus einer biologischen Probe entfernt werden, bevor Mikrovesikel isoliert und aufgereinigt und/oder angereichert werden. Aus den Mikrovesikeln waren dann Nukleinsäuren hoher Qualität extrahiert. Bei dem beschriebenen Verfahren wird eine biologische Probe bereitgestellt und mit einer Einfangfläche in Kontakt gebracht, unter Bedingungen, die ausreichen, um zellfreie DNA (Desoxyribonukleinsäure) und Mikrovesikel aus der biologischen Probe auf oder in der Einfangfläche zu erhalten. Die Einfangfläche wird mit einem Phenol-basierten Lysereagenz in Kontakt gebracht, während zellfreie DNA und die Mikrovesikel auf oder in der Einfangfläche sind, wodurch DNA und RNA (Ribonukleinsäuren) aus der Probe freigesetzt werden und ein Homogenat produziert wird. Danach werden DNA, RNA oder sowohl DNA als auch RNA aus dem Homogenat extrahiert.

Ein ähnliches Verfahren zur Isolierung von extrazellulären Vesikeln und Co-Isolierung von zellfreier DNA aus Bioflüssigkeiten ist aus der US 10,808,240 B2 bekannt. Eine biologische Probe wird mit einer festen Einfangoberfläche unter Bedingungen in Kontakt gebracht, die ausreichen, um zellfreie DNA und Mikrovesikel aus der biologischen Probe auf oder in der Einfangoberfläche zurückzuhalten. Die Einfangoberfläche wird mit einem Elutionspuffer auf GTC-Basis in Kontakt gebracht, während sich zellfreie DNA und die Mikrovesikel auf oder in der Einfangoberfläche befinden, wodurch die DNA und RNA aus der Probe freigesetzt und ein Homogenat erzeugt wird. Die DNA, die RNA, oder sowohl die DNA als auch die RNA werden aus dem Homogenat extrahiert.

Auch aus der EP 2 245 458 B1 ist ein Verfahren bekannt, bei dem ein Homogenat erzeugt wird, das aus DNA aus extrazellulären Vesikeln und cfDNA besteht.

Aus der US 2014/030788 A1 sind Vorrichtungen und Systeme mit Fluidkanälen bzw. Fluidkammern und darin angeordneten permeablen Hindernissen bekannt, die jeweils eine Mehrzahl von ausgerichteten Nanostrukturen aufweisen, die eine äußere Oberfläche des Hindernisses und ein inneres Netzwerk von Hohlräumen definieren. Die Hindernisse ermöglichen das Einfangen von Partikeln eines ersten Typs in einer Fluidprobe oder die selektive Separation oder Konzentration von Partikeln des ersten Typs von Partikeln eines zweiten Typs. Partikel des ersten Typs können z.B. Krebszellen sein und Partikel des zweiten Typs können z.B. Mikrovesikel oder Nukleinsäure sein.

### Beschreibung der Erfindung

Es wurde erkannt, dass ein wesentlicher Nachteil der im Stand der Technik bekannten Verfahren darin besteht, dass nach Durchführung dieser Verfahren die EV-DNA und die cfDNA (zusätzlich je nach Verfahren RNA) als Homogenat vorliegen und nicht differenziert analysiert werden können. Die extrazellulären Vesikel werden zerstört, d.h. Merkmale, wie z.B. Oberflächenmarker, Größenverteilung usw., können nicht weiter untersucht werden. Extrazelluläre Vesikel enthalten weitere relevante Analyten (z.B. Proteine). Diese gehen bei den bekannten Verfahren, bei denen ein Homogenat erzeugt wird, verloren. Die Menge an cfDNA stellt bereits einen Biomarker dar. Diese Menge wird durch zusätzliche EV-DNA verfälscht.

Es wurde ferner erkannt, dass Abhilfe ein Analysieren verschiedener Analyt-Klassen aus dem identischen Probenvolumen schaffen könnte, wobei mehrere verschiedene Analyten die diagnostische Sensitivität/Spezifität steigern können, was jedoch bisher ein großes Probenvolumen erforderte. Vorzugsweise sollten die angewandten Verfahren zur prä-analytischen Isolation der verschiedenen Analyt-Klassen zum einen kompatibel miteinander sein und zum anderen in einer integrierten, automatisierten Prozesskette vereint werden können. Dadurch könnte eine wirtschaftlich attraktive Lösung geboten werden, da hohe Kosten durch manuelle Zwischenschritte und außerdem Bedarf nach mehreren verschiedenen Laborgeräten zur Isolation vermieden werden könnten. Zum aktuellen Zeitpunkt liegt kein Verfahren vor, welches es ermöglicht, mehrere Analyt-Klassen aus einem identischen Probenvolumen und in einer einzelnen integrierten Prozesskette zu isolieren, da die bestehenden Verfahren sich entweder gegenseitig beeinflussen/stören würden und/oder auf unterschiedlichen, nicht ohne weiteres in einem Laborgerät kombinierbaren, Prinzipien basieren.

Es ist die Aufgabe der Erfindung, Verfahren und Vorrichtungen zu schaffen, die es ermöglichen Analyten unterschiedlicher Analyt-Klassen, nämlich extrazelluläre Vesikel und/oder zirkulierenden Tumorzellen auf der einen Seite und zellfreie Nukleinsäuren auf der anderen Seite aus dem gleichen Probenvolumen auf eine Weise zu isolieren, die eine Implementierung in einer integrierten Prozesskette ermöglicht.

Diese Aufgabe wird durch ein Verfahren nach Anspruch 1 und eine zentrifugal mikrofluidische Vorrichtung nach Anspruch 6 gelöst. Weiterbildungen der Erfindung sind in den abhängigen Ansprüchen definiert.

Beispiele der Offenbarung schaffen Verfahren zum Isolieren von Analyten einer ersten Analyt-Klasse, die extrazelluläre Vesikel und/oder zirkulierende Tumorzellen sind, und Analyten einer zweiten Analyt-Klasse, die zellfreie Nukleinsäuren sind, aus dem gleichen Probenvolumen einer biologischen Probe in einem zentrifugal-mikrofluidischen System, das ein Fluidikmodul aufweist, welches eine erste Isolationskammer, die eine erste Isolationsstruktur enthält, und eine zweite Isolationskammer, die eine zweite Isolationsstruktur enthält, aufweist. Das Probenvolumen wird in die erste Isolationskammer geleitet, so dass die Analyten der ersten Analyt-Klasse durch die erste Isolationsstruktur zurückgehalten werden, während die Analyten der zweiten Analyt-Klasse nicht von der ersten Isolationsstruktur zurückgehalten werden und als Teil einer Restflüssigkeit die erste Isolationsstruktur passieren. Die Restflüssigkeit wird in die zweite Isolationskammer geleitet, so dass die Analyten der zweiten Analyt-Klasse durch die zweite Isolationsstruktur zurückgehalten werden. Die Analyten der ersten Analyt-Klasse werden von der ersten Isolationsstruktur getrennt und die Analyten der zweiten Analyt-Klasse werden von der zweiten Isolationsstruktur getrennt, um die Analyten der ersten Analyt-Klasse und die Analyten der zweiten Analyt-Klasse separat voneinander für eine nachfolgende Analyse bereitzustellen. Die erste Isolationsstruktur ist ein Filter mit einer Porengröße in einem Bereich von 20 Nanometern bis 200 Nanometern, vorzugsweise in einem Bereich von 20 Nanometern bis 45 Nanometern. Die zweite Isolationsstruktur ist eine Oberfläche zum Binden von Analyten der zweiten Analyt-Klasse ist, wobei die Oberfläche durch Partikel gebildet ist, und wobei die Partikel magnetisierbar sind. Das Verfahren weist ein Drehen des Fluidikmoduls auf, um mittels eines oder mehrerer stationärer oder verfahrbarer Magneten eines Prozessierungsgeräts die magnetisierbaren Partikel in der zweiten Isolationskammer zu bewegen, um ein Durchmischen der Partikel mit der Restflüssigkeit zu unterstützen, und/oder bei einem Transfer von Analyten der zweiten Analyt-Klasse in die zweite Sammelkammer ein Zurückhalten der magnetisierbaren Partikel in der zweiten Isolationskammer zu unterstützen.

Beispiele der Offenbarung schaffen eine Vorrichtung mit einem Prozessierungsgerät und Fluidikmodul zum Isolieren von Analyten einer ersten Analyt-Klasse, die extrazelluläre Vesikel und/oder zirkulierende Tumorzellen sind, und Analyten einer zweiten Analyt-Klasse, die zellfreie Nukleinsäuren sind, aus dem gleichen Probenvolumen einer biologischen Probe in einem zentrifugal-mikrofluidischen System. Das Fluidikmodul weist eine erste Isolationskammer, welche eine erste Isolationsstruktur enthält, eine zweite Isolationskammer, welche eine zweite Isolationsstruktur enthält, eine erste Fluidleitung, eine zweite Fluidleitung, eine erste Sammelkammer und eine zweite Sammelkammer auf. Die erste Fluidleitung ist ausgelegt, um das Probenvolumen in die erste Isolationskammer zu leiten, so dass die Analyten der ersten Analyt-Klasse durch die erste Isolationsstruktur zurückgehalten werden, während die Analyten der zweiten Analyt-Klasse nicht von der ersten Isolationsstruktur zurückgehalten werden und als Teil einer Restflüssigkeit die erste Isolationsstruktur passieren. Die zweite Fluidleitung ist ausgelegt, um die Restflüssigkeit in die zweite Isolationskammer zu leiten, so dass die Analyten der zweiten Analyt-Klasse durch die zweite Isolationsstruktur zurückgehalten werden. Die erste Sammelkammer ist mit der ersten Isolationskammer verbunden und ausgelegt, um von der ersten Isolationsstruktur gelöste Analyten der ersten Analyt-Klasse aufzunehmen. Die zweite Sammelkammer ist mit der zweiten Isolationskammer verbunden und ist ausgelegt, um von der zweiten Isolationsstruktur gelöste Analyten der zweiten Analyt-Klasse aufzunehmen, oder um die zweite Isolationsstruktur aufzunehmen, nachdem die Analyten der zweiten Analyt-Klasse davon gelöst wurden. Die erste Isolationsstruktur ist ein Filter mit einer Porengröße in einem Bereich von 20 Nanometern bis 200 Nanometern, vorzugsweise in einem Bereich von 20 Nanometern bis 45 Nanometern. Die zweite Isolationsstruktur ist eine Oberfläche zum Binden von Analyten der zweiten Analyt-Klasse ist, und wobei die Oberfläche durch magnetisierbare Partikel gebildet ist. Das Prozessierungsgerät weist einen oder mehrere stationäre oder verfahrbare Magneten auf, die außerhalb der zweiten Isolationskammer angeordnet sind, um bei einer Rotation des Fluidikmoduls ein Durchmischen magnetisierbarer Partikel mit dem Filtrat zu unterstützen und/oder um bei einem Transfer von Analyten der zweiten Analyt-Klasse in die zweite Sammelkammer ein Zurückhalten der magnetisierbaren Partikel in der zweiten Isolationskammer zu unterstützen.

Gemäß Beispielen der Offenbarung werden somit Analyten unterschiedlicher Analyt-Klassen aus dem gleichen Probenvolumen gewonnen und separat voneinander bereitgestellt, für eine weitere Verwendung derselben, beispielsweise eine Analyse oder Untersuchung derselben. Da beide Analyten aus dem gleichen Probenvolumen gewonnen werden, wobei die Analyten der zweiten Analyt-Klasse aus der nach der Isolation der Analyten der ersten Analyt-Klasse verbliebenen Restflüssigkeit gewonnen werden, ist eine Isolierung beider Analyt-Klassen aus einem kleinen Probenvolumen möglich. Ferner wurde erkannt, dass es möglich ist, beide Isolierungen unter Verwendung eines Fluidikmoduls in einem zentrifugalen mikrofluidischen System durchzuführen, so dass eine Integration in eine einzelne Prozesskette möglich ist.

### Kurze Beschreibung der Zeichnungen

Ausführungsbeispiele der Erfindung werden nachfolgend Bezug nehmend auf die beigefügten Zeichnungen näher erläutert. Es zeigen:
Fig. 1 eine schematische Darstellung eines Fluidikmoduls gemäß einem Beispiel der vorliegenden Offenbarung;
Fig. 2 eine schematische Darstellung von Komponenten eines Fluidikmoduls gemäß einem Beispiel der vorliegenden Offenbarung;
Fig. 3 ein Diagramm, das schematisch ein Verfahren gemäß der vorliegenden Offenbarung zeigt; und
Fig. 4 eine schematische Darstellung eines Fluidikmoduls in Form einer zentrifugal-mikrofluidischen Kartusche gemäß einem Beispiel der vorliegenden Offenbarung;
Fig. 5A und 5B schematische Darstellungen von Vorrichtungen, die Fluidikmodule, wie sie hierin beschreiben sind, verwenden.

### Detaillierte Beschreibung

Im Folgenden werden Beispiele der vorliegenden Offenbarung detailliert und unter Verwendung der beigefügten Zeichnungen beschrieben. Es sei darauf hingewiesen, dass gleiche Elemente oder Elemente, die die gleiche Funktionalität aufweisen, mit gleichen oder ähnlichen Bezugszeichen versehen sind, wobei eine wiederholte Beschreibung von Elementen, die mit dem gleichen oder ähnlichen Bezugszeichen versehen sind, typischerweise weggelassen wird. Insbesondere können gleiche oder ähnliche Elemente jeweils mit Bezugszeichen versehen sein, die eine gleiche Zahl mit einem unterschiedlichen oder keinem Kleinbuchstaben aufweisen. Beschreibungen von Elementen, die gleiche oder ähnliche Bezugszeichen aufweisen, können gegeneinander austauschbar sein. In der folgenden Beschreibung werden viele Details beschrieben, um eine gründlichere Erklärung von Beispielen der Offenbarung zu liefern. Es ist jedoch für Fachleute offensichtlich, dass andere Beispiele ohne diese spezifischen Details implementiert werden können. Merkmale der unterschiedlichen beschriebenen Beispiele können miteinander kombiniert werden, es sei denn Merkmale einer entsprechenden Kombination schließen sich gegenseitig aus oder eine solche Kombination ist ausdrücklich ausgeschlossen.

Bevor Beispiele der vorliegenden Offenbarung näher erläutert werden, werden Definitionen einiger hierin verwendeter Begriffe angegeben.

Unter dem Begriff Isolation werden hierin Verfahren verstanden, die das Ziel haben, die störenden Bestandteile einer Probenmatrix, z.B. Inhibitoren oder fälschlicherweise analysierbare Komponenten, zu entfernen und/oder die Konzentration eines Analyten in einer Probe erhöhen, z.B. durch Verringerung des wässrigen Anteils einer Probe. Dabei kann die Isolation entweder durch Anreicherung des Analyten, oder durch Abreicherung von Nicht-Analyt-Stoffen geschehen. Synonyme Begriffe für den Begriff Isolation sind Anreicherung, Extraktion und Aufreinigung.

Unter einer Probe wird in diesem Kontext ein biologisches Material bezeichnet, welches zumeist von einem Menschen stammt und mittels einer nicht- oder minimal-invasiven Technik gesammelt wird. Dabei kann die Probe sowohl eine flüssige Probe (z.B. Vollblut, Blut-plasma, Blutserum, Urin, Speichel, Tränenflüssigkeit, Zerebrospinalflüssigkeit, Seminalplasma) als auch eine verflüssigte Probe festen Ursprungs (z.B. Stuhl) sein. Ebenso fallen unter diese Definition Proben, welche auf Basis einer menschlichen Zellprobe generiert wurden, z.B. ein Zellkultur-Überstand, auch wenn die ursprüngliche Zellprobe invasiv gesammelt wurde.

Unter einer Probenmatrix werden die Bestandteile einer Probe verstanden, welche nicht analysiert werden sollen. Dabei kann die Matrix eine Analyse erschweren, z.B. wenn der Analyt in nur sehr geringer Konzentration vorliegt oder wenn Bestandteile der Matrix das Analyseverfahren stören.

Unter Analyt werden Bestandteile verstanden, die analysiert werden sollen. In der Probenmatrix gelöste Analyten sind u.a. Stoffe, Moleküle, Partikel, welche in einem an die Isolation angeschlossenen Analyseverfahren hinsichtlich Anzahl, Konzentration, Biomarker-Signaturen, etc. untersucht werden können. Mögliche Analyten in diesem Kontext sind u.a. Nukleinsäuren, Proteine, Peptide, Metabolite, Sekundärmetabolite, Vitamine, Zellen (humane Zellen, sowie Pilze, Bakterien oder Mykoplasmen), Exosome sowie Viren.

Unter dem Begriff Analyt-Klassen werden hier diejenigen Analyten zusammengefasst, welche mit einem einzelnen technischen Verfahren isoliert werden können. In diesem Kontext wird z.B. DNA und RNA in ihren verschiedensten Ausprägungen als eine Analyt-Klasse betrachtet, nämlich zellfreie Nukleinsäuren (cfNA). Die heterogenen Subtypen von extrazellulären Vesikeln werden ebenfalls als eine Analyt-Klasse betrachtet, nämlich extrazelluläre Vesikel (EV). Diese Analyt-Klasse kann ferner oder alternativ zirkulierende Tumorzellen umfassen. Dabei ist zu bedenken, dass je nach Ausprägung des Analyten von Interesse, beispielsweise kleine EV gegenüber großen EV, die Parameter des Isolationsverfahrens variieren können, beispielsweise variierende Porengrößen bei einer Filtration. Weitere Analyt-Klassen können nach dieser Definition Proteine, Zellen und Zellfragmente sein.

Unter dem Begriff zellfreier Nukleinsäuren (cell-free nucleic acids, cfNA) werden hier verschiedene Formen von DNA und RNA zusammengefasst, welche außerhalb von Zellen im menschlichen Körper vorkommen. Nach aktuellem Stand umfasst dies u.a. zirkulierende zellfreie DNA (ccfDNA) sowohl nukleären als auch mitochondrialen Ursprungs, zirkulierende Tumor-DNA (ctDNA), zellfreie DNA (cfDNA), zelluläre DNA, zirkulierende Tumor-RNA (ctRNA), microRNA (miRNA), und non-coding RNA (ncRNA).

Unter dem Begriff extrazelluläre Vesikel (EV) werden von Zellen freigesetzte Partikel zusammengefasst. Sie stellen eine heterogene Population aus verschiedenen Subtypen dar, welche nach aktuellem Stand Exosome, Mikrovesikel, Ectosome und apoptotische Körperchen umfassen. Die Größe der Partikel variiert je nach Subtyp zwischen einigen 10 nm und einigen 1 µm. Sowohl im Inneren der EV, als auch zum Teil an deren Oberfläche, befinden sich verschiedenen Analyten, wie z.B. Proteine, Nukleinsäuren, Lipide oder Metabolite.

Als Proteine werden Makromoleküle bezeichnet, welche aus Aminosäuren bestehen, die mittels Peptidbindungen verknüpft sind. Sie übernehmen verschiedenste Funktionen im menschlichen Körpern und sind an einer Vielzahl von krankheits-assoziierten Prozessen beteiligt. Die Analyse von zellfreien Proteinen in einer Probenmatrix, z.B. Blutserum, kann deshalb alleinig oder komplementär mit anderen Analyten zur Diagnostik verwendet werden.

Unter dem Begriff Zellen bzw. Zellfragmente wird hier die Gesamtheit aller menschlichen Blutzellen (Erythrozyten, Leukozyten und Thrombozyten), sowie weitere zirkulierende (z.B. im Blut) bzw. transportierte (z.B. im Stuhl) Zellen zusammengefasst. Ein Beispiel für zirkulierende Zellen im Blut sind Zirkulierende Tumorzellen (Circulating Tumor Cells, CTC) oder Tumor assoziierte Thrombozyten (Tumor Educated Platelets, TEP).

Unter einem Fluidikmodul ist hierein ein Modul, beispielsweise eine Kartusche zu verstehen, das Mikrofluidikstrukturen aufweist, die ausgelegt sind, um eine Flüssigkeitshandhabung, wie sie hierin beschrieben ist, zu ermöglichen. Unter einem zentrifugalen mikrofluidischen Fluidikmodul (Kartusche) ist ein entsprechendes Modul zu verstehen, das einer Rotation unterworfen werden kann, beispielsweise in Form eines in einen Rotationskörper einsetzbaren Fluidikmoduls oder eines Rotationskörpers.

Beispiele der Erfindung können insbesondere auf dem Gebiet der zentrifugalen Mikrofluidik Anwendung finden, bei der es um die Prozessierung von Flüssigkeiten im Picoliter- bis Milliliterbereich geht. Entsprechend können die Fluidikstrukturen geeignete Abmessungen im Mikrometerbereich für die Handhabung entsprechender Flüssigkeitsvolumina aufweisen.

Wird hierin der Ausdruck radial verwendet, so ist jeweils radial bezüglich des Rotationszentrums, um das das Fluidikmodul bzw. der Rotationskörper drehbar ist, gemeint. Im Zentrifugalfeld ist somit eine radiale Richtung von dem Rotationszentrum weg radial abfallend und eine radiale Richtung zu dem Rotationszentrum hin ist radial ansteigend. Ein Fluidkanal, dessen Anfang näher am Rotationszentrum liegt als dessen Ende, ist somit radial abfallend, während ein Fluidkanal, dessen Anfang weiter vom Rotationszentrum entfernt ist als dessen Ende, radial ansteigend ist. Ein Kanal, der einen radial ansteigenden Abschnitt aufweist, weist also Richtungskomponenten auf, die radial ansteigen bzw. radial nach innen verlaufen. Es ist klar, dass ein solcher Kanal nicht exakt entlang einer radialen Linie verlaufen muss, sondern in einem Winkel zu der radialen Linie oder gebogen verlaufen kann.

Ist hierin nichts anderes angegeben, ist hinsichtlich temperaturabhängiger Größen jeweils von Raumtemperatur (20°C) auszugehen.

Allgemein beziehen sich Beispiele der vorliegenden Offenbarung auf Verfahren und Vorrichtungen zur Aufreinigung mehrerer Analyten aus einem Probenvolumen. Beispiele beziehen sich dabei auf ein Verfahren zur Isolation von mehreren unterschiedlichen Klassen von Analyten, beispielsweise zellfreien Nukleinsäuren und extrazellulären Vesikeln, kurz EV, aus einem einzigen Probenvolumen, wobei die Probe typischerweise von einem Menschen stammt und mittels einer nicht- oder minimal-invasiven Technik gesammelt wird, beispielsweise Blut, Urin oder Stuhl. Beispiele ermöglichen dies ohne das Probenvolumen in mehrere Subvolumina aufzuteilen, da mit einer solchen Aufteilung ein Verlust an absoluter Analyt-Menge pro Analyse einhergeht, was wiederum in einem Verlust an erreichbarer Sensitivität resultiert. Weiterhin soll vermieden werden, dass verschiedene Analyt-Klassen gemeinsam in ein einziges Isolat isoliert werden, da auf diese Weise die strukturell gleichen Bestandteile der verschiedenen Analyten, beispielsweise der zellfreien Nukleinsäuren und der EV-assoziierten Nukleinsäuren, nicht differenziert voneinander analysiert werden können. Als geeignetes Verfahren zur Vermeidung der Proben-Aufteilung wurde eine zentrifugal-mikrofluidische Prozesskette identifiziert, die eine Isolation von mehreren Klassen von Analyten aus dem identischen Probenvolumen ermöglicht, wobei die einzelnen Isolationsverfahren miteinander kompatibel sind und sich nicht gegenseitig störend beeinflussen. Im Gegensatz zu bisherigen Verfahren, welche entweder eine Aufteilung der Probe vor der Isolation oder mehreren seriellen Isolationsverfahren erfordern, ermöglichen die hier beschriebenen Verfahren eine automatisierte Isolation mehrerer Analyten in einer einzigen integrierten Prozesskette. Als Produkte des Prozesses entstehen mehrere Isolate, die jeweils nur eine einzelne definierte Klasse von Analyten enthalten.

Beispiele der Offenbarung schaffen somit Verfahren zur Isolation von zwei oder mehr Analyt-Klassen aus einem einzelnen Probenvolumen in einem kombinierten, integrierten Prozess in einer zentrifugal-mikrofluidischen Kartusche sowie Fluidikmodule in Form von Rotationskörpern und Vorrichtungen, die ausgelegt sind, um solche Verfahren auszuführen. Ziel ist es dabei, Probenmaterial und Zeit einzusparen und den manuellen sowie Instrumentationsaufwand zu minimieren und gleichzeitig möglichst viele Analyten in hoher Reinheit für nachfolgende Analysen zu isolieren.

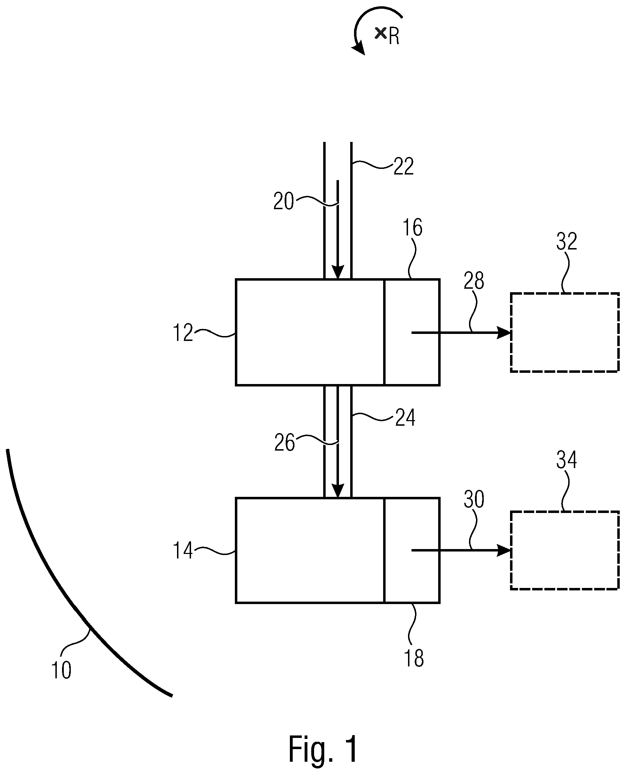

Fig. 1 zeigt schematisch ein Beispiel eines Fluidikmoduls 10 gemäß der vorliegenden Offenbarung, das ausgelegt ist, um ein Verfahren zur Isolation von zwei Analyt-Klassen durchzuführen, wie es hierin beschrieben ist. Das Fluidikmodul 10 kann beispielsweise ein um ein Rotationszentrum R drehbarer Rotationskörper oder ein in einen solchen Rotationskörper einsetzbares Fluidikmodul sein. Das Fluidikmodul 10 weist Fluidikstrukturen auf, die eine erste Isolationskammer 12 und eine zweite Isolationskammer 14 aufweisen. Die erste Isolationskammer 12 enthält eine erste Isolationsstruktur 16. Die zweite Isolationskammer 14 enthält eine zweite Isolationsstruktur 18. Ein Probenvolumen wird in die erste Isolationskammer 12 geleitet, wie durch einen Pfeil 20 in Fig. 1 gezeigt ist. Zu diesem Zweck weisen die Fluidikstrukturen eine erste Fluidleitung 22 auf. Analyten der ersten Analyt-Klasse werden durch die erste Isolationsstruktur 16 zurückgehalten, während Analyten der zweiten Analyt-Klasse nicht von der ersten Isolationsstruktur 16 zurückgehalten werden, sondern als Teil einer Restflüssigkeit die erste Isolationsstruktur 16 passieren. Die Restflüssigkeit wird durch eine zweite Fluidleitung 24, die die erste Isolationskammer 12 mit der zweiten Isolationskammer 14 verbindet, in die zweite Isolationskammer 14 geleitet, wie durch einen Pfeil 26 in Fig. 1 gezeigt ist. Die Analyten der zweiten Analyt-Klasse werden durch die zweite Isolationsstruktur 18 zurückgehalten.

Die Analyten der ersten Analyt-Klasse werden von der ersten Isolationsstruktur 16 getrennt und die Analyten der zweiten Analyt-Klasse werden von der zweiten Isolationsstruktur 18 getrennt. Dies ist durch Pfeile 28 und 30 in Fig. 1 gezeigt. Dadurch werden die Analyten der ersten Analyt-Klasse und die Analyten der zweiten Analyt-Klasse separat voneinander für eine nachfolgende Analyse bereitgestellt. Das Trennen 28 der Analyten der ersten Analyt-Klasse von der ersten Isolationsstruktur kann beispielsweise ein Leiten der Analyten der ersten Analyt-Klasse in eine erste Sammelkammer 32 aufweisen. Die erste Sammelkammer 32 kann dazu über eine Fluidleitung mit der ersten Isolationskammer 12 verbunden sein. Das Trennen 30 der Analyten der zweiten Analyt-Klasse von der zweiten Isolationsstruktur 18 kann ein Leiten der Analyten der zweiten Analyt-Klasse in eine zweite Sammelkammer, die mit der ersten Sammelkammer über eine Fluidleitung verbunden ist, umfassen. Die erste Sammelkammer 32, die zweite Sammelkammer 34 und Fluidleitungen, die dieselben mit der ersten Isolationskammer 12 beziehungsweise der zweiten Isolationskammer 14 verbinden, können Teil der Fluidikstrukturen des Fluidikmoduls 10 sein. Die erste Sammelkammer 32 ist somit zur Aufnahme von von der ersten Isolationsstruktur 16 gelösten Analyten der ersten Analyt-Klasse ausgelegt, und die zweite Sammelkammer 34 ist zur Aufnahme von von der zweiten Isolationsstruktur 18 gelösten Analyten der zweiten Analyt-Klasse ausgelegt.

Bei Beispielen des beschriebenen Verfahrens spielt die Reihenfolge, in der die Schritte des Trennens der Analyten der ersten Analyt-Klasse von der ersten Isolationsstruktur und des Leitens der Restflüssigkeit in die zweite Isolationskammer stattfinden, keine Rolle. Bei Beispielen kann zunächst die Restflüssigkeit in die zweite Isolationskammer geleitet werden, bevor die Analyten der ersten Analyt-Klasse von der ersten Isolationsstruktur getrennt werden. Bei Beispielen der vorliegenden Offenbarung ist die erste Isolationsstruktur ausgelegt, um eine Filtration aufgrund von Größenunterschieden durchzuführen. Bei Beispielen ist die erste Isolationsstruktur 16 ein Filter mit einer Porengröße in einem Bereich von 20 Nanometern bis 200 Nanometern, vorzugsweise in einem Bereich von 20 Nanometern bis 45 Nanometern. Somit ermöglicht die erste Isolationsstruktur die Isolation von Analyten der ersten Analyt-Klasse, nämlich extrazellulären Vesikeln und/oder zirkulierenden Tumorzellen. Alternativ könnten durch eine solche Filtration auch Thrombozyten isoliert werden.

Bei Beispielen weist die erste Isolationsstruktur Fängerstrukturen auf, die ausgelegt sind, um Analyten der ersten Analyt-Klasse zu binden. Solche Fängerstrukturen können beispielsweise Antikörper oder Aptamere sein. Alternativ können affinitätsbasierte Methoden angewendet werden, zum Beispiel Peptide auf magnetisierbaren Partikeln, die Bestandteile der EV-Membran binden. Bei Beispielen kann die erste Isolationsstruktur ausgelegt sein, um eine Polymerfällung zu bewirken. Dabei wird ein Polymer zugegeben, welches ein Gitter bildet, in dem die EVs beziehungsweise zirkulierenden Tumorzellen (CTC = Circulating Tumor Cell) eingefangen werden. Anschließend kann eine Sedimentation durchgeführt werden, um das Gitter abzutrennen, das heißt die Analyten der ersten Analyt-Klasse von der ersten Isolationsstruktur zu trennen.

Bei Beispielen kann zum Trennen der Analyten der ersten Analyt-Klasse von der ersten Isolationsstruktur ein Waschen der an der ersten Isolationsstruktur zurückgehaltenen Analyten mittels einer Waschlösung, ein Eluieren der Analyten von der ersten Isolationsstruktur mittels einer Eluierungslösung und ein Transferieren der von der ersten Isolationsstruktur gelösten Analyten in eine erste Sammelkammer aufweisen. Somit ermöglichen Beispiele ein Isolieren und Trennen der Analyten der ersten Analyt-Klasse auf eine gängige Art und Weise.

Bei Beispielen weist die erste Isolationsstruktur eine volumetrisch definiere Kammergeometrie der ersten Isolationskammer auf, zur Sedimentation von Analyten der ersten Analyt-Klasse. Die geometrisch definierte Kammergeometrie kann beispielsweise durch einen Kanal gegeben sein, der auf einer bestimmten radialen Höhe in die erste Isolationskammer mündet. Durch eine Rotation kann eine Sedimentation in der ersten Isolationskammer bewirkt werden, durch die die Analyten der ersten Analyt-Klasse in den Kammerbereich, der radial außerhalb des in die Kammer mündenden Kanals liegt, gelangen. Die Restflüssigkeit kann dann in die zweite Isolationskammer geleitet werden, indem der Überstand über den in der bestimmten radialen Höhe in die erste Isolationskammer mündenden Kanal abgezogen wird. Die erste Isolationsstruktur ist in diesem Fall durch den unteren Teil der ersten Isolationskammer gebildet. Bei Beispielen können EVs in einem Polymer, das die erste Isolationsstruktur bildet, absedimentiert werden, woraufhin der Überstand abgenommen werden kann, ein Lösepuffer hinzugefügt werden kann, der das Polymer wieder auflöst, und anschließend kann die Lösung mit den EVs transferiert werden. In einem solchen Fall kann der Kanal zum Abziehen des Überstands unter Umständen am radial äußeren Ende der ersten Isolationskammer angebracht sein, um die Restflüssigkeit abzuziehen, wobei das Polymer mit den Analyten der ersten Analyt-Klasse in der ersten Isolationskammer verbleibt.

Bei Beispielen können die Isolationsstrukturen, insbesondere die zweite Isolationsstruktur, eine Oberfläche zum Binden von Analyten sein. Die Oberfläche kann durch Partikel, Säulen, eine Membran oder eine Oberfläche des Fluidikmoduls gebildet sein. Eine solche Isolationsstruktur kann eine Festphasenisolation (bind, wash, elute = binden, waschen eluieren) aufgrund von kontrollierten Bindungsbedingungen an eine Oberfläche bewirken, beispielsweise eine Isolation von zellfreien Nukleinsäuren oder eine Isolation von Proteinen. Dabei kann die Oberfläche sowohl Teil des Fluidikmoduls, der Kartusche, sein, beispielweise ein Kanal oder eine Kammer, oder kann ein in das Fluidikmodul eingebrachter Körper oder eine Vielzahl von in das Fluidikmodul eingebrachten Körpern sein, beispielsweise Säule/Membran oder Beads/Nanopartikel).

Bei Beispielen weist zumindest die zweite Isolationsstruktur eine Oberfläche zum Binden von Analyten der zweiten Analyt-Klasse auf die durch Partikel, beispielsweise Beads, gebildet ist, wobei die Partikel magnetisierbar sind, wobei das Verfahren ein Drehen des Fluidikmoduls aufweist, um die magnetisierbaren Partikel in der zweiten Isolationskammer mittels einer oder mehrerer stationärer oder verfahrbarer Magneten eines Prozessierungsgeräts zu bewegen, um ein Durchmischen der Partikel mit der Flüssigkeit in der jeweiligen Kammer, beispielsweise mit der Restflüssigkeit, zu unterstützen. Die Magnete können bei Beispielen nicht nur zur Durchmischung dienen, sondern auch ausgelegt sein, um Partikel in der zweiten Isolationskammer zurückzuhalten, wenn die Eluierungsflüssigkeit mit dem Analyten in die Sammelkammer transferiert wird. Bei Beispielen können somit außerhalb der zweiten Isolationskammer ein oder mehrere Magnete stationär oder verfahrbar angeordnet sein, um beim Transfer der Analyten der zweiten Analyt-Klasse in die zweite Sammelkammer ein Zurückhalten der magnetisierbaren Partikel in der zweiten Isolationskammer zu unterstützen.

Beispiele der vorliegenden Offenbarung schaffen eine zentrifugale mikrofluidische Vorrichtung, die ein Fluidikmodul, wie es hierin beschrieben ist, und ein Prozessierungsgerät aufweist, das ausgelegt ist, um das Fluidikmodul mit einer Rotation zu beaufschlagen. Das Prozessierungsgerät kann ein oder mehrere stationäre oder verfahrbare Magneten aufweisen, die bei Aktuierung des Fluidikmoduls, das heißt bei einer Drehung desselben, magnetisierbare Partikel in der zweiten Isolationskammer bewegen, um ein Durchmischen der Partikel in der Restflüssigkeit zu unterstützen.

Bei Beispielen der hierin offenbarten Verfahren umfasst das Isolieren der Analyten der zweiten Analyt-Klasse ein Mischen des Probenvolumens mit einem Bindepuffer, das Inkontaktbringen des dadurch erhaltenen Gemisches mit einer Oberfläche, um die Analyten der zweiten Analyt-Klasse an der Oberfläche zu binden, ein Waschen der Oberfläche mit den gebundenen Analyten unter Verwendung eines Waschpuffers, ein Eluieren der Analyten von der Oberfläche unter Verwendung eines Elutionspuffers, und ein Transferieren der von der zweiten Isolationsstruktur gelösten Analyten in eine zweite Sammelkammer. Bei Beispielen kann ein vergleichbares Verfahren verwendet werden, um die Analyten der ersten Analyt-Klasse aus dem Probenvolumen zu isolieren, wobei die gelösten Analyten in eine erste Sammelkammer transferiert werden können. Beispiele ermöglichen somit eine Isolation der Analyten der ersten und/oder zweiten Analyt-Klasse unter Verwendung von gängigen Bind-Wash-Elute-Verfahren.

Bei Beispielen der vorliegenden Offenbarung kann eine Isolation von Analyten aus dem Probenvolumen mittels Zentrifugation erfolgen. Durch eine solche Zentrifugation kann vor, zwischen oder nach einer Filtration eine weitere Auftrennung der Probe aufgrund von Dichteunterschieden realisiert werden, beispielsweise zur Blut-Plasma-Separation oder zur Isolation von Thrombozyten. Beispiele der vorliegenden Offenbarung sind ausgelegt, um Analyten von zwei oder mehr Analyt-Klassen aus dem Probenvolumen zu extrahieren. Sollen drei oder mehr Analyten isoliert werden, so sind die Fludikstrukturen entsprechend ausgelegt und weisen zumindest eine weitere Isolationskammer mit zumindest einer weiteren Isolationsstruktur und zumindest einer weiteren Sammelkammer auf. Die verschiedenen Analyt-Klassen können anschließend aus den verschiedenen Sammelkammern des Fluidikmoduls, der mikrofluidischen Kartusche, für Analysen entnommen werden.

Bei Beispielen weist das Fluidikmodul zumindest ein Filter auf, das mit der Eingangsseite der ersten Isolationskammer fluidisch verbunden ist, das für die Analyten der ersten Analyt-Klasse und die Analyten der zweiten Analyt-Klasse durchlässig ist, und das ausgelegt ist, um Zellen, Zellfragmente, Partikel und Verunreinigung aus dem Probenvolumen zu filtern. Entsprechend weisen Beispiele des Verfahrens vor dem Leiten des Probenvolumens in die erste Isolationskammer ein Leiten des Probenvolumens durch ein entsprechendes vorgeschaltetes Filter auf. Somit ermöglichen Beispiele eine Vorbereitung des Probenvolumens für die spätere Isolation der Analyten der ersten Analyt-Klasse und zweiten Analyt-Klasse aus dem Probenvolumen.

Bei Beispielen der vorliegenden Offenbarung weist das Vorbereiten der biologischen Probe einen oder mehrere der folgenden Prozesse auf:
- Durchführen einer Blut-Plasma-Separation der biologischen Probe, um Plasma zu erhalten, aus dem das Probenvolumen gewonnen wird.
- Durchführen einer Blut-Plasma-Separation einer koagulierten biologischen Probe, um Serum zu erhalten, aus dem das Probenvolumen gewonnen wird.
- Durchführen einer Thrombozyten-Isolation des Plasmas, um Thrombozyten-armes Plasma zu erhalten, aus dem das Probenvolumen gewonnen wird.
- Durchführen einer Thrombozyten-Isolation des Plasmas, um Thrombozyten-reiches Plasma zu erhalten, aus dem das Probenvolumen gewonnen wird.
- Durchführen eines enzymatischen Verdaus, um Proteine und Proteinaggregate in der biologischen Probe abzubauen, um das Probenvolumen zu gewinnen.
- Durchführen einer Verflüssigung und/oder Homogenisierung der biologischen Probe, um das Probenvolumen zu gewinnen.

Bei Beispielen weist das Fluidikmodul Fluidikstrukturen auf, die ausgelegt sind, um einen oder mehrerer der vorher genannten Prozesse durchzuführen.

Eine Thrombozyten-Isolation kann beispielsweise mittels einer Sedimentation durchgeführt werden, wobei das Sediment Thrombozyten-reichem Plasma und der Überstand Thrombozyten-armem Plasma entspricht. Abhängig davon, ob man das Probenvolumen mittels eines Kanals an einem radial unteren Ende oder einem radial seitlichen Ende der Sedimentationskammer entnimmt, kann somit Thrombozyten-armes oder Thrombozyten-reiches Plasma gewonnen werden. Ferner kann eine Thrombozyten-Isolation mittels einer Filtration erreicht werden, indem das Fluidikmodul ein Filter aufweist, dessen Porengröße ausgelegt ist, um die Thrombozyten zurückzuhalten. Das Filtrat wäre dann Thrombozyten-arm und das Retentat beziehungsweise anschließend vom Filter gelöste Eluat wäre Thrombozytenreich. Die Größe von Thrombozyten beträgt ca. 1 bis 4 µm. Es ist beispielsweise bekannt, zum Zurückhalten von Thrombozyten Filter mit einer entsprechenden Porengröße, beispielsweise einer Porengröße von 600 Nanometern, zu verwenden.

Bei Beispielen der vorliegenden Offenbarung ist die erste Isolationsstruktur ausgelegt, um EVs und/oder CTCs zurückzuhalten, wobei das Trennen der Analyten von der ersten Isolationsstruktur ein Trennen der EVs und/oder CTCs im Ganzen oder in ihre Bestandteile zerlegt von der Isolationsstruktur umfasst. Bei Beispielen können EVs/CTCs bei gleichzeitigem Verbleiben der cfDNA im Durchfluss durch eines der folgenden Verfahren abgetrennt werden: - Filtration, - Methoden, bei denen die Analyten eingefangen werden ("capture"), wobei beispielweise Antikörper, Aptamere zum Einsatz kommen oder affinitätsbasierte Methoden angewendet werden, wie zum Beispiel Peptide auf magnetisierbaren Partikeln, die Bestandteile der EV-Membran binden; - Polymerfällung, wo ein Polymer zugegeben wird, welches ein Gitter bildet, in dem die EVs/CTCs eingefangen werden, wobei anschließend sedimentiert wird, um das Gitter abzutrennen; - Sedimentation, um CTCs aus dem Probenvolumen zu isolieren.

Generell kann bei Beispielen des hierin beschriebenen Verfahrens eine Probe mit einem Volumen von wenigen Mikrolitern bis mehreren Millilitern automatisiert oder manuell in das Fluidikmodul (die mikrofluidische Kartusche) transferiert werden. Das Fluidikmodul wird anschließend in einem Gerät automatisch weiter prozessiert. Dabei werden aus der Probe sukzessive, in einem einzigen Prozess über verschieden Verfahren, Analyten mehrerer Analyt-Klassen isoliert. Zu diesem Zweck kann das Gerät ausgelegt sein, um das Fluidikmodul Rotationen zu unterwerfen, durch die die verschiedenen Flüssigkeiten durch die Fluidikstrukturen bewegt werden, wie es hierin beschrieben ist. Bei Beispielen kann das Fluidmodul dazu mit einem entsprechenden Frequenzprotokoll beaufschlagt werden, so dass die in den Kartuschen befindlichen Flüssigkeiten durch die Zentrifugalkraft bewegt werden können. Die Fluidikstrukturen können ferner ausgelegt sein, um die Handhabung der Flüssigkeiten durch weitere Prozesse, beispielsweise hydrodynamische Prozesse, zu unterstützen. Die Probe wird dabei nicht für die jeweiligen Isolationsverfahren aufgeteilt, sondern sie wird mittels mikrofluidischer Verbindungskanäle automatisiert weitergereicht, so dass die jeweiligen Analyten aus dem kompletten Probenvolumen isoliert werden.

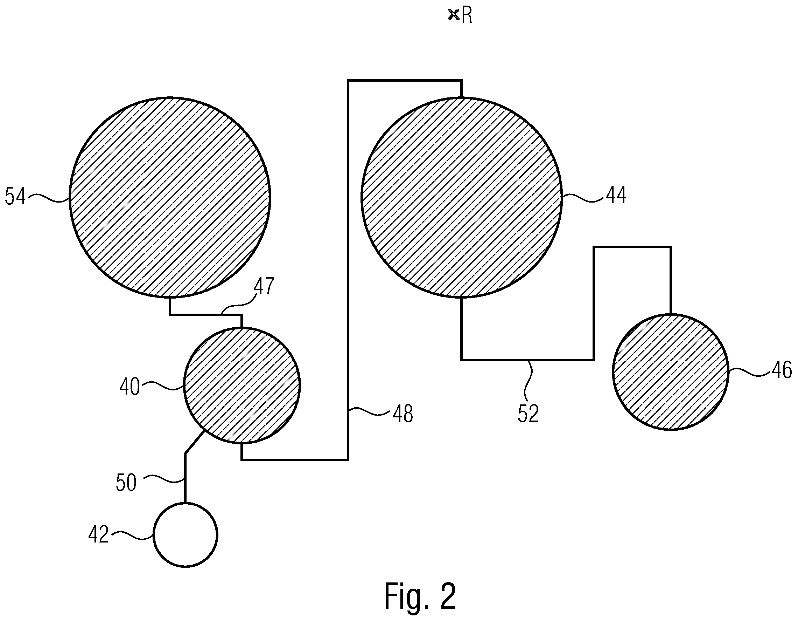

Fig. 2 zeigt schematisch Komponenten eines Fluidikmoduls gemäß einem Beispiel der vorliegenden Offenbarung. Die Komponenten weisen eine erste Filterstruktur 40, die eine erste Isolationsstruktur darstellt, eine erste Auffangstruktur 42, die eine erste Sammelkammer darstellt, eine Separationsstruktur 44, die eine zweite Isolationsstruktur darstellt, und eine zweite Auffangstruktur 46, die eine zweite Sammelkammer darstellt, auf. Eine erste Fluidleitung 47 führt zu der ersten Filterstruktur 40, eine zweite Fluidleitung 48 verbindet die Filterstruktur 40 mit der Separationsstruktur 44, eine dritte Fluidleitung 50 verbindet die erste Auffangstruktur 42 mit der Filterstruktur 40 und eine vierte Fluidleitung 52 verbindet die zweite Auffangstruktur 46 mit der Separationsstruktur 44. Wie in Fig. 2 gezeigt ist, kann bei Beispielen optional eine weitere Filterstruktur 54 vorgeschaltet sein, die eine Porengröße aufweist, die extrazelluläre Vesikel, zirkulierende Tumorzellen und zellfreie Nukleinsäuren passieren lässt. Die erste Filterstruktur 40 ist ausgelegt, um extrazelluläre Vesikel zurückzuhalten und zellfreie Nukleinsäuren passieren zu lassen. Bei Beispielen kann die erste Filterstruktur ausgelegt sein, um zirkulierende Tumorzellen zurückzuhalten. Bei alternativen Beispielen kann die erste Filterstruktur durch eine Isolationsstruktur ersetzt sein, die ausgelegt ist, um zirkulierende Tumorzellen durch Sedimentation zurückzuhalten.

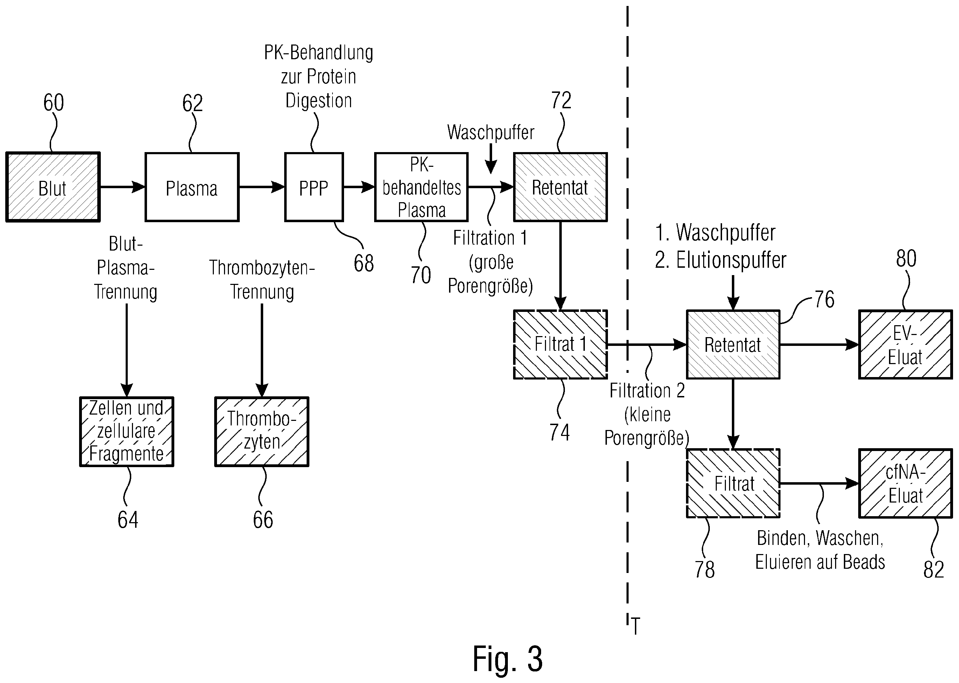

Bezugnehmend auf Fig. 3 wird nachfolgend eine mögliche Implementierung eines Ablaufs zur Aufreinigung mehrerer Analyten aus einem Probenvolumen erläutert. Dabei sind in Fig. 3 rechts von einer Trennlinie T die Schritte dargestellt, die tatsächlich das Verfahren zur Isolierung zweier unterschiedlicher Analyt-Klassen aus einem Probenvolumen betreffen, während links von der Trennlinie T ein Verfahren zur Vorbereitung des Probenvolumens dargestellt ist. Beispiele von Fluidikmodulen der vorliegenden Offenbarung weisen Fluidikstrukturen auf, um das in Fig. 3 gezeigte Verfahren durchzuführen. Das Verfahren muss jedoch nicht alle in Fig. 3 gezeigten Prozesse aufweisen, wobei insbesondere die links der Trennlinie T gezeigten Prozessschritte ganz oder teilweise als optional angesehen werden können.

Eine Blutprobe wird in das Fluidikmodul transferiert, 60. Eine Blut-Plasma-Trennung wird durchgeführt, um Plasma zu erhalten, 62, wobei Zellen und zelluläre Fragmente abgetrennt werden, 64. Nachfolgend kann eine Thrombozyten-Trennung erfolgen, um Thrombozyten aus dem Plasma abzutrennen, 66. Im Anschluss kann ein Proteinverdau durch Proteinase K (PK) zur Protein-Digestion durchgeführt werden, 68. Dadurch wird PK-behandeltes Plasma erhalten, 70. Das so erhaltene Plasma kann nun einer groben Filterung mit einer großen Porengröße unterworfen werden, beispielsweise durch eine Filterstruktur 54, wie sie in Fig. 2 gezeigt ist. Durch diese Filtration, die in Fig. 3 als Filtration 1 bezeichnet ist, wird ein Retentat 72 und ein Filtrat 74 erhalten. Das Filtrat 74 stellt das Probenvolumen der biologischen Probe dar, aus dem zunächst EV und dann NA isoliert wird. Die primäre Auftrennung erfolgt bei dem gezeigten Beispiel mittels einer Filtration, die in Fig. 3 als Filtration 2 bezeichnet ist. Dabei wird die optional zuvor aufbereitete Probe mithilfe von Zentrifugalkräften durch eine oder mehrere Filtermembranen gepresst. Die Filtermembranen können aus unterschiedlichen Materialien bestehen und unterschiedliche Porengrößen aufweisen, beispielsweise zwischen 20 nm und 10 µm. Mögliche Materialien für die Membranen sind Polyester (PES), Recyclingmaterial (RC), anodisiertes Aluminiumoxid (AAO), oder TEPC (gewebeäquivalentes Material). Werden mehrere Filtermembranen eingesetzt, wird die Probe nacheinander durch die verschiedenen Filtermembranen gepresst, zunächst Filtermembranen mit größerem Porendurchmesser und nachfolgend Filtermembranen mit kleinerem Porendurchmesser. Beispielsweise können durch die vorgeschaltete Filtration 1 Matrixbestandteile, zum Beispiel Zellen, Zellfragmente oder Proteinaggregate, mit großem Durchmesser abgetrennt werden, wohingegen die EV und cfNA den Filter, beispielsweise die weitere Filterstruktur 54 in Fig. 2, passieren. Das finale Filter, das die erste Isolationsstruktur darstellt, weist eine solche Porengröße auf, dass kleinere Kontaminationen, beispielsweise einzelne Proteine, und die cfNA die Membran passieren, die EV jedoch zurückgehalten werden. Diese Filtration ist in Fig. 3 als Filtration 2 bezeichnet. Durch diese Filtration 2 wird ein Retentat 76 und ein Filtrat 78 erhalten. Das Filtrat 78 stellt die Restflüssigkeit dar, die zu der zweiten Isolationsstruktur geleitet wird.

Um die Matrixkomponenten im Retentat 76 weiter abzureichern, können Waschschritte mittels wässriger Lösungen, Waschpuffern, erfolgen. Die so gereinigten EV können dann für weitere Analysen mittels eines Elutionspuffers vom Filter getrennt und als EV-Eluat 80 in eine Sammelkammer des Fluidikmoduls transferiert werden. Der Durchfluss des Filters, das heißt das Filtrat 78, beinhaltet neben der verbleibenden Probenmatrix auch die cfNA. Diese kann nun über weitere Methoden konzentriert und gereinigt werden. Hierfür stehen auf zentrifugal-mikrofluidischen Kartuschen, dem Fluidikmodul, verschiedene Methoden zur Verfügung, beispielsweise eine cfNA-Festphasenisolation an Beads. Bei einer solchen Festphasenisolation wird cfNA an Beads gebunden, danach mittels eines Waschpuffers gewaschen und unter Verwendung eines Eluierungspuffers eluiert und als cfNA-Eluat 82 in eine Sammelkammer des Fluidikmoduls transferiert. Somit werden Analyten der ersten Analyt-Klasse und Analyten der zweiten Analyt-Klasse separat voneinander für eine nachfolgende Analyse bereitgestellt.

Bei Beispielen, bei denen die zweite Isolationsstruktur aus der zweiten Isolationskammer bewegt werden kann, kann nach dem Trennen der Analyten der zweiten Analyt-Klasse von der zweiten Isolationsstruktur die zweiten Isolationsstruktur aus der zweiten Isolationskammer bewegt werden, während die Analyten der zweiten Analyt-Klasse in der zweiten Isolationskammer verbleiben. Bei dem obigen Beispiel könnte beispielsweise die Festphasenmatrix in Form der Beads nach dem Eluieren aus der Isolationskammer transferiert werden, während das Eluat in der Isolationskammer verbleibt.

Wie oben bereits ausgeführt wurde, kann unter Ausnutzung der Zentrifugalkräfte im zentrifugal-mikrofluidischen System optional bei Blutproben eine Blut-Plasma-Separation vorgeschaltet werden. Diese kann zum einen den manuellen Aufwand zur Probenvorbereitung weiter reduzieren und zum anderen die Reproduzierbarkeit des gesamten analytischen Prozesses weiter steigern. Im Gegensatz zur manuellen Blut-Plasma-Separation kann durch eine zentrifugal-pneumatische Operation der Plasma-Überstand nach Zentrifugation von Vollblut unter Rotation abgenommen werden, wodurch Re-Sedimentationseffekte praktisch vermieden werden. Als weiteres Ziel einer integrierten, vorgeschalteten Blut-Plasma-Separation kann die dabei automatisch erreichte Isolation von Zellen und Zellfragmenten betrachtet werden, die als eine weitere Klasse von Analyten betrachtet werden kann. Dabei kann neben der Analyse von gesunden Zellen insbesondere die Analyse krankheits-assoziierter Zellen von Relevanz sein, wie beispielsweise zirkulierender Tumorzellen, CTCs.

Bei Beispielen stellt die Sedimentation von zirkulierenden Tumor-Zellen keine Vorbehandlung der biologischen Probe dar, sondern ein Isolieren von Analyten der zweiten Analyt-Klasse. Bei anderen Beispielen stellt das Isolieren der zirkulierenden Tumorzellen das Isolieren von Analyten einer dritten Analyt-Klasse dar, das dem Isolieren von EV als Analyten einer ersten Analyt-Klasse und dem Isolieren von cfNA als Analyten einer zweiten Analyt-Klasse vorgeschaltet ist.

Analog zur Blut-Plasma-Separation kann unter Anwendung der inhärenten Zentrifugalkraft, sowie einem ein- oder mehrfachen Abnehmen des Überstandes eine Separation von Plasma in Thrombozyten-reiches Plasma (PRP = Platelet Rich Plasma) und Thrombozyten-armes Plasma (PPP = Platelet Poor Plasma) erreicht werden. Das dabei erzeugte PRP kann als Isolat zur Analyse von Thrombozyten aus der Kartusche entnommen werden, und beispielsweise zur Analyse von Tumor Educated Platelets (TEP) genutzt werden. Das erzeugte PPP kann hingegen in den weiteren Prozess der sukzessiven Isolation der Analyt-Klassen EV und cfNA gehen. Wenn zellfreie DNA mitochondrialen Ursprungs (cf-mtDNA) analysiert werden soll, kann eine solche vorgeschaltete Thrombozyten-Isolation essentiell sein, um die Verunreinigung der zellfreien mitochondrialen DNA mit mitochondrialer DNA aus lysierten Thrombozyten (mtDNA) zu verhindern beziehungsweise zu minimieren. TEPs können hierbei als Analyten einer dritten Analyt-Klasse betrachtet werden, die vor den Analyten der ersten und zweiten Analyt-Klassen aus dem Probenvolumen entnommen werden.

Weiterhin optional und je nach Matrix kann vor der Filtration eine Verflüssigung und/oder Homogenisierung der biologischen Probe, beispielsweise bei Sputum- oder Stuhlproben, oder ein enzymatischer Verdau mit Protease (beispielsweise Proteinase K) erfolgen. Hierbei werden Proteine und Proteinaggregate abgebaut, die eine Isolation der Analyten erschweren, beispielsweise indem sie die Filtermembranen verstopfen oder Nukleinsäuren binden. Die Enzyme können durch Waschschritte, beispielsweise den in Fig. 3 angedeuteten Waschpuffer, beziehungsweise eine Festphasenisolation (bind-wash-elute) wieder von den Analyten getrennt werden, so dass sie die weiteren Analysen nicht behindern.

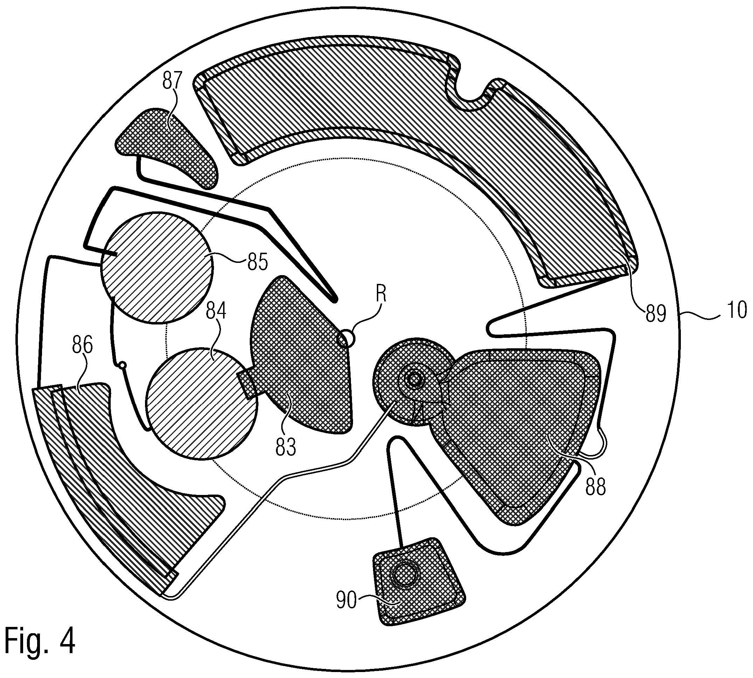

Fig. 4 zeigt schematisch ein Fluidikmodul 10 in Form eines um ein Rotationszentrum R drehbaren Rotationskörpers 10, der Fluidikstrukturen aufweist, die ausgelegt sind, um eine automatisierte, kombinierte Isolation von cfNA und EV aus einem einzigen Probenvolumen in Form einer humanen Plasmaprobe durchzuführen. In dem Fluidikmodul gebildete Fluidikstrukturen weisen eine Probeneinlasskammer 83, eine Vorfiltrationskammer 84, eine Filtrationskammer 85 zur EV-Isolation, eine Kammer 86 zum Auffangen des Filtrats aus der Filtrationskammer 85, eine Sammelkammer 87 zum Entnehmen des EV-Isolats, eine Kammer 88 zur Festphasenisolation von cfNA, eine Sammelkammer 89 für Waschflüssigkeiten der Festphasenisolation, und eine Sammelkammer 90 zum Entnehmen des cfNA-Isolats auf. Wie in Fig. 4 gezeigt ist, sind die jeweiligen Kammern durch Fluidleitungen miteinander verbunden. Die Fluidleitungen sind ausgestaltet, um einen Transfer von Flüssigkeit zwischen den jeweiligen Kammern unter Ausnutzung der Zentrifugalkraft und möglicherweise anderer Effekte, beispielsweise hydrodynamischer Effekte, zu ermöglichen. Die Filtrationskammer 85 zur EV-Isolation stellt eine erste Isolationskammer, wie sie hierin beschrieben ist, dar und die Kammer 88 stellt eine zweite Isolationskammer, wie sie hierhin beschrieben ist, dar.

Eine Filtermembran kann dabei jeweils einen einlassseitigen Abschnitt der Fluidkammer, in der die Filtermembran angeordnet ist, von einem auslassseitigen Abschnitt dieser Fluidkammer trennen. Bei Beispielen sind der einlassseitige Abschnitt der Fluidkammer und der auslassseitige Abschnitt der Fluidkammer in Richtung einer durch das Rotationszentrum verlaufenden Rotationsachse übereinander angeordnet.

Im Betrieb wird die Plasmaprobe in die Probeneinlasskammer 83 gegeben und anschließend sequenziell durch die in den Kammern 84 und 85 angeordneten Filtermembranen filtriert. Die in der Kammer 84 angeordnete Filtermembran hat dabei größere Porendurchmesser als die in der Kammer 85 angeordnete Filtermembran, so dass die Analyten der ersten und zweiten Analytklasse passieren können, größere Matrixbestandteile aber zurückgehalten werden. Die zweite Filtermembran in der Kammer 85 ist so konzipiert, dass cfNA passieren können, die EV aber zurückgehalten werden. So können beide Analyten aus der Probe verlustfrei voneinander getrennt werden. Das cfNA-Filtrat wird in der Kammer 86 gesammelt und von dort weiter in die Kammer 88 zur Festphasenisolation transferiert. Alternativ könnte das Filtrat auch direkt in die Kammer zur Festphasenisolation transferiert werden. Die auf der zweiten Filtermembran in der Kammer 85 zurückgehaltenen EV werden anschließend mit wässrigen Pufferlösungen gewaschen, die manuell oder automatisiert in die Kammer 83 zugegeben werden können, um Matrixbestandteile zu entfernen, die die Filter passieren können. Die Waschlösungen können dann in der Kammer 86 gesammelt werden, nachdem das cfNA-Filtrat in die Kammer 88 transferiert wurde. Nach dem Waschen der EV werden diese von dem Filter in der Kammer 85 in die Sammelkammer 87 transferiert, aus der sie manuell oder automatisiert entnommen werden können. Das Filtrat mit den cfNA wird in Kammer 88 einer Festphasenisolation unterworfen, indem es mit einer Oberfläche in Kontakt gebracht wird. Bei Beispielen erfolgt eine Festphasenisolation nach dem Bind-Wash-Elute-Verfahren unter Verwendung magnetischer bzw. magnetisierbarer Partikel, mit denen das Filtrat in Kontakt gebracht wird, die die NA über eine Silikaoberfläche binden. In einem oder mehreren Waschschritten mit lösungsmittelhaltigen Flüssigkeiten können Matrixbestandteile, wie zum Beispiel Proteine, entfernt werden. Die Waschlösungen werden in der Kammer 89 gesammelt. Nach dem Waschen werden die cfNA mithilfe eines wässrigen Elutionspuffers von den magnetisierbaren Partikeln eluiert und anschließend zur Entnahme in die Kammer 90 transferiert.

Verglichen mit dem Stand der Technik bei seriellen Einzelverfahren, bei denen beispielsweise eine Isolation von extrazellulären Vesikeln mittels Filtration in einer ersten Apparatur, dann ein manueller Transfer und eine anschließende Isolation von Nukleinsäuren mittels einer Festphasenisolation in einer zweiten Apparatur stattfindet, ermöglicht das hierin vorgestellte Kombinationsverfahren die Isolation verschiedener Analyten nicht nur in einer einzelnen Apparatur, einem zentrifugal-mikrofluidischen Prozessiergerät, sondern zudem in einem einzigen Verbrauchsartikel, nämlich einem Fluidikmodul in Form einer zentrifugal-mikrofluidischen Kartusche. Neben Vorteilen durch eine einfache Handhabung und geringere Material- und Personalkosten, ist zu erwarten, dass sich diese integrierte Prozesskette vorteilhaft auf die Reproduzierbarkeit der Isolation auswirkt. Dieser Aspekt ist von höchster Relevanz, da prä-analytische Variationen als eine wesentliche Hürde bei der klinischen Translation von zirkulierenden Analyten betrachtet wird. Im Gegensatz zum Stand der Technik bei Kombinationsverfahren ermöglicht das vorgestellte Verfahren die Isolation verschiedener Analyt-Klassen aus einem einzigen Probenvolumen, ohne eine negative Beeinflussung der jeweiligen Analyten. Konkret lassen sich bei dem hierin beschriebenen Verfahren die Analyt-Klasse der extrazellulären Vesikel von der Analyt-Klasse der Nukleinsäuren abtrennen und jeweils aus der Probenmatrix isolieren, ohne dass eine der beiden Analyt-Klassen in signifikantem Maße zerstört, beschädigt oder minimiert wird. Die dabei erzeugten, qualitativ hochwertigen Isolate der jeweiligen Analyt-Klassen ermöglichen genaue und reproduzierbare Analysen auf Einzel- und Multi-Analyt-Ebene zur Steigerung von Sensitivität und Spezifität, ohne eine unterwünschte Steigerung des Probenvolumens.

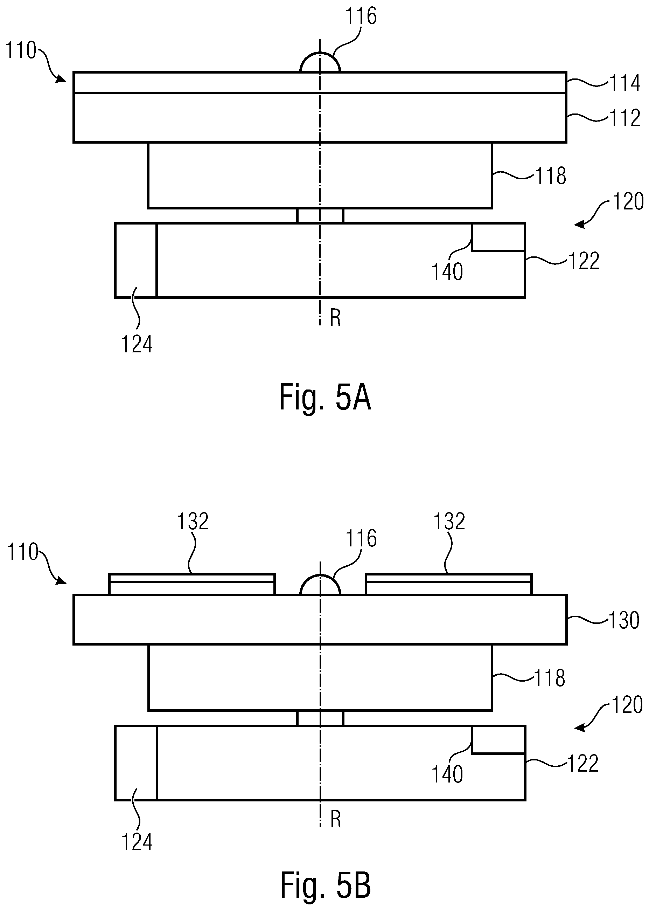

Bezugnehmend auf die Figuren 5A und 5B werden nun Beispiele von zentrifugal-mikrofluidischen Systemen beschrieben, die ein Fluidikmodul, wie es hierin beschrieben ist, verwenden bzw. aufweisen. Mit anderen Worten kann das Fluidikmodul bei den Systemen in den Figuren 5A und 5B ein beliebiges der hierein beschrieben Fluidikmodule sein.

Fig. 5A zeigt eine Vorrichtung mit einem Fluidikmodul 110 in Form eines Rotationskörpers, der ein Substrat 112 und einen Deckel 114 aufweist. Das Substrat 112 und der Deckel 114 können in Draufsicht kreisförmig sein, mit einer mittigen Öffnung, über die der Rotationskörper 110 über eine übliche Befestigungseinrichtung 116 an einem rotierenden Teil 118 einer Antriebsvorrichtung 120 angebracht sein kann. Das rotierende Teil 118 ist drehbar an einem stationären Teil 122 der Antriebsvorrichtung 120 gelagert. Bei der Antriebsvorrichtung 120 kann es beispielsweise um eine herkömmliche Zentrifuge, die eine einstellbare Drehgeschwindigkeit aufweisen kann, oder auch ein CD- oder DVD-Laufwerk handeln. Eine Steuereinrichtung 124 kann vorgesehen sein, die ausgelegt ist, um die Antriebsvorrichtung 120 zu steuern, um den Rotationskörper 110 mit einer Rotation oder mit Rotationen unterschiedlichen Drehfrequenzen zu beaufschlagen. Die Steuereinrichtung 124 kann, wie für Fachleute offensichtlich ist, beispielsweise durch eine entsprechend programmierte Recheneinrichtung oder eine anwenderspezifische integrierte Schaltung implementiert sein. Die Steuereinrichtung 124 kann ferner ausgelegt sein, um auf manuelle Eingaben durch einen Benutzer hin die Antriebsvorrichtung 120 zu steuern, um die erforderlichen Rotationen des Rotationskörpers zu bewirken. In jedem Fall kann die Steuereinrichtung 124 konfiguriert sein, um die Antriebsvorrichtung 120 zu steuern, um den Rotationskörper mit der erforderlichen Rotation zu beaufschlagen, um Ausführungsbeispiele der Erfindung, wie sie hierin beschrieben sind, zu implementieren. Als Antriebsvorrichtung 120 kann eine herkömmliche Zentrifuge mit nur einer Drehrichtung verwendet werden.

Der Rotationskörper 110 weist die erforderlichen Fluidikstrukturen auf. Die erforderlichen Fluidikstrukturen können durch Kavitäten und Kanäle in dem Deckel 114, dem Substrat 112 oder in dem Substrat 112 und dem Deckel 114 gebildet sein. Bei Ausführungsbeispielen können beispielsweise Fluidikstrukturen in dem Substrat 112 abgebildet sein, während Einfüllöffnungen und Entlüftungsöffnungen in dem Deckel 114 gebildet sind. Bei Ausführungsbeispielen ist das strukturierte Substrat (inklusive Einfüllöffnungen und Entlüftungsöffnungen) oben angeordnet und der Deckel unten angeordnet.

Bei einem alternativen in Fig. 5B gezeigten Ausführungsbeispiel sind Fluidikmodule 132 in einen Rotor 130 eingesetzt und bilden zusammen mit dem Rotor 130 den Rotationskörper 110. Die Fluidikmodule 132 können jeweils ein Substrat und einen Deckel aufweisen, in denen wiederum entsprechende Fluidikstrukturen gebildet sein können. Der durch den Rotor 130 und die Fluidikmodule 132 gebildete Rotationskörper 110 ist wiederum durch eine Antriebsvorrichtung 120, die durch die Steuereinrichtung 124 gesteuert wird, mit einer Rotation beaufschlagbar.

In den Figuren 5A und 5B ist das Rotationszentrum, um das das Fluidikmodul bzw. der Rotationskörper drehbar ist, wiederum mit R bezeichnet.

Die in den Figuren 5A und 5B gezeigte Vorrichtung stellt ein Prozessierungsgerät dar, das ausgelegt ist, um das Fluidikmodul mit den für die Durchführung der hierein beschriebenen Verfahren erforderlichen Rotationen zu beaufschlagen. Das Prozessierungsgerät kann einen oder mehrere stationäre oder verfahrbare Magneten 140 aufweisen, die ausgelegt sind, um ein Durchmischen von Partikeln mit der Restflüssigkeit in der zweiten Isolationskammer zu unterstützen. Zu diesem Zweck können die Magneten 140 an einer geeigneten Stelle des stationären Teils der Antriebsvorrichtung 120 angebracht sein, die die zweite Isolationskammer passiert, so dass das Magnetfeld derselben auf magnetisierbare Partikel in der zweiten Isolationskammer einwirken kann.

Bei Ausführungsbeispielen der Erfindung können das Fluidikmodul bzw. der Rotationskörper, das bzw. der die Fluidikstrukturen aufweist, aus einem beliebigen geeigneten Material gebildet sein, beispielsweise einem Kunststoff, wie PMMA (Polymethylmethacrylat), PC (Polycarbonat), PVC (Polyvinylchlorid) oder PDMS (Polydimethylsiloxan), Glas oder dergleichen. Der Rotationskörper 110 kann als eine zentrifugal-mikrofluidische Plattform betrachtet werden. Bei bevorzugten Ausführungsbeispielen können das Fluidikmodul bzw. der Rotationskörper aus einem Thermoplast, wie z.B. PP (Polypropylen), PC, COP (Cyclic Olefin Polymer), COC (Cyclo Olefin Copolymer) oder PS (Polystyrol) gebildet sein.

Hierin beschriebene Fluidikmodule ermöglichen somit, dass mehrere Analyt-Klassen aus einem identischen Probenvolumen isoliert werden und in separaten Isolaten aus dem Prozess entnommen werden können. Bisher wurde zur Erzeugung mehrerer Isolate mit jeweils nur einer Analyt-Klasse entweder eine Fraktionierung der Probe in mehrere Subvolumina durchgeführt, was einen Verlust an Sensitivität zur Folge hat, oder es wurde ein größeres Probenvolumen fraktioniert, um die gleiche Sensitivität zu erreichen, was in der Praxis jedoch aufgrund der Patientenbelastung oder der limitierten Probenressourcen nicht realisierbar ist. Es ist in der zentrifugalen Mikrofluidik keine Technologie bekannt, die sämtliche Prinzipien zur Realisierung der Isolation verschiedener Analyt-Klassen bietet (Zentrifugation, Abnahme von Überstand, Ein- und Mehrstufen-Filtration und Festphasenisolation) und deren Kombination in integrierte, automatisierte Prozessketten ermöglicht. Es wurde insbesondere überraschend erkannt, dass sich die einzelnen Schritte der hier vorgestellten Prozesskette, nämlich der EV-Filtration und der cfNA-Festphasenisolation sowie verschiedene optionale Schritte, nicht in signifikantem Maße gegenseitig negativ beeinflussen. Beispielsweise wurde überraschend erkannt, dass es bei einer Aufreinigung von EV mittels Filtration nicht zu einem signifikanten Verlust von cfNA durch Absorption an der Filteroberfläche beziehungsweise im Filtervolumen kommt.

Beispiele der vorliegenden Offenbarung schaffen einen Rotationskörper, bei dem eine oder mehrere in Serie geschaltete Filterstrukturen zum Filtern integriert sind, die mithilfe von Zentrifugalkraft durchströmt werden können und deren Filterporen einen Durchmesser zwischen 20 nm und 200 nm aufweisen, und bei dem eine Auffangstruktur für den Durchfluss vorgesehen ist. Die Auffangstruktur kann eine Isolationsstruktur enthalten, um den Durchfluss mit einem Puffer zu mischen und nachfolgend das Puffer-Durchfluss-Gemisch mit einer (aktiven) Oberfläche in Kontakt zu bringen, um Nukleinsäuren an dieser Oberfläche zu binden. Weiterhin kann der Rotationskörper eine integrierte weitere fluidische Struktur aufweisen, um das Puffer-Durchfluss-Gemisch aus der Separationsstruktur in der weiteren fluidischen Struktur aufzufangen, und auch mindestens einen Waschpuffer, der die (aktive) Oberfläche wäscht, aufzufangen. Die (aktive) Oberfläche wird aus magnetisierbaren Partikeln gebildet. Ein Magnet ist in das Gerät zur Beaufschlagung der Rotation integriert, der verfahrbar sein kann und die Durchmischung der Beads mit der Flüssigkeit unterstützen kann. Bei Beispielen kann die aktive Oberfläche eine Silikamembran sein.

Bei Beispielen kann die zweite Isolationsstruktur, wie sie hierin beschrieben ist, eine Separationsstruktur sein, wie sie in der DE 10 2018 219 091 A1 beschrieben ist. Bei Beispielen können die erste und zweite Isolationsstruktur durch Isolationsstrukturen gebildet sein, wie sie für sich genommen bekannt und eingangs beschrieben sind.

Es bedarf keiner separaten Erläuterung, dass hierin beschriebene Kammern, wie Isolationskammern und Fluidkammern, jeweils mehrere Kammerabschnitte aufweisen können oder durch mehrere Kammern gebildet sein können. Kammern, beispielsweise Fluidkammern oder Isolationskammern, die über eine Fluidleitung verbunden sind, müssen nicht direkt über die Fluidleitung verbunden sein, sondern weitere Fluidkammern können dazwischen angeordnet sein.

Obwohl Merkmale der Erfindung jeweils anhand von Vorrichtungsmerkmalen oder Verfahrensmerkmalen beschrieben wurden, ist für Fachleute offensichtlich, dass entsprechende Merkmale jeweils auch Bestandteil eines Verfahrens oder einer Vorrichtung sein können. So kann jeweils die Vorrichtung konfiguriert sein, um entsprechende Verfahrensschritte durchzuführen, und die jeweilige Funktionalität der Vorrichtung kann entsprechende Verfahrensschritte darstellen

In der vorhergehenden detaillierten Beschreibung wurden teilweise verschiedene Merkmale in Beispielen zusammen gruppiert, um die Offenbarung zu rationalisieren. Diese Art der Offenbarung soll nicht als die Absicht interpretiert werden, dass die beanspruchten Beispiele mehr Merkmale aufweisen als ausdrücklich in jedem Anspruch angegeben sind. Vielmehr kann, wie die folgenden Ansprüche wiedergeben, der Gegenstand in weniger als allen Merkmalen eines einzelnen offenbarten Beispiels liegen. Folglich werden die folgenden Ansprüche hiermit in die detaillierte Beschreibung aufgenommen, wobei jeder Anspruch als ein eigenes separates Beispiel stehen kann. Während jeder Anspruch als ein eigenes separates Beispiel stehen kann, sei angemerkt, dass, obwohl sich abhängige Ansprüche in den Ansprüchen auf eine spezifische Kombination mit einem oder mehreren anderen Ansprüchen zurückbeziehen, andere Beispiele auch eine Kombination von abhängigen Ansprüchen mit dem Gegenstand jedes anderen abhängigen Anspruchs oder einer Kombination jedes Merkmals mit anderen abhängigen oder unabhängigen Ansprüchen umfassen. Solche Kombinationen seien umfasst, es sei denn es ist ausgeführt, dass eine spezifische Kombination nicht beabsichtigt ist. Ferner ist beabsichtigt, dass auch eine Kombination von Merkmalen eines Anspruchs mit jedem anderen unabhängigen Anspruch umfasst ist, selbst wenn dieser Anspruch nicht direkt abhängig von dem unabhängigen Anspruch ist.

Die oben beschriebenen Beispiele sind nur darstellend für die Grundsätze der vorliegenden Offenbarung. Es ist zu verstehen, dass Modifikationen und Variationen der Anordnungen und der Einzelheiten, die beschrieben sind, für Fachleute offensichtlich sind. Es ist daher beabsichtigt, dass die Offenbarung nur durch die beigefügten Patentansprüche und nicht durch die spezifischen Einzelheiten, die zum Zwecke der Beschreibung und Erklärung der Beispiele dargelegt sind, begrenzt ist.

## Patentansprüche

1. Verfahren zum Isolieren von Analyten einer ersten Analyt-Klasse, die extrazelluläre Vesikel und/oder zirkulierende Tumorzellen sind, und Analyten einer zweiten Analyt-Klasse, die zellfreie Nukleinsäuren sind, aus dem gleichen Probenvolumen einer biologischen Probe in einem zentrifugal-mikrofluidischen System, das ein Fluidikmodul (10, 110, 132) aufweist, der eine erste Isolationskammer (12, 85), welche eine erste Isolationsstruktur (16, 40) enthält, und eine zweite Isolationskammer (14, 88) welche eine zweite Isolationsstruktur (18, 44) enthält, aufweist, mit folgenden Merkmalen:
Leiten des Probenvolumens in die erste Isolationskammer (12, 85), so dass die Analyten der ersten Analyt-Klasse durch die erste Isolationsstruktur (16, 40) zurückgehalten werden, während die Analyten der zweiten Analyt-Klasse nicht von der ersten Isolationsstruktur (16, 40) zurückgehalten werden und als Teil einer Restflüssigkeit die erste Isolationsstruktur (16, 40) passieren;
Leiten der Restflüssigkeit in die zweite Isolationskammer (14, 88), so dass die Analyten der zweiten Analyt-Klasse durch die zweite Isolationsstruktur (18, 44) zurückgehalten werden; und
Trennen der Analyten der ersten Analyt-Klasse von der ersten Isolationsstruktur (16, 40) und Trennen der Analyten der zweiten Analyt-Klasse von der zweiten Isolationsstruktur (18, 44), um die Analyten der ersten Analyt-Klasse und die Analyten der zweiten Analyt-Klasse separat voneinander für eine nachfolgende Analyse bereitzustellen,
wobei die erste Isolationsstruktur (16, 40) ein Filter mit einer Porengröße in einem Bereich von 20 Nanometern bis 200 Nanometern, vorzugsweise in einem Bereich von 20 Nanometern bis 45 Nanometern ist,
wobei die zweite Isolationsstruktur (18, 44) eine Oberfläche zum Binden von Analyten der zweiten Analyt-Klasse ist, wobei die Oberfläche durch Partikel gebildet ist, und
wobei die Partikel magnetisierbar sind, wobei das Verfahren ein Drehen des Fluidikmoduls (10, 110, 132) aufweist, um mittels eines oder mehrerer stationärer oder verfahrbarer Magneten (140) eines Prozessierungsgeräts die magnetisierbaren Partikel in der zweiten Isolationskammer (14, 88) zu bewegen, um ein Durchmischen der Partikel mit der Restflüssigkeit zu unterstützen, und/oder bei einem Transfer von Analyten der zweiten Analyt-Klasse in die zweite Sammelkammer ein Zurückhalten der magnetisierbaren Partikel in der zweiten Isolationskammer (14, 88) zu unterstützen.

2. Verfahren nach Anspruch 1, das ein Waschen der an der ersten Isolationsstruktur (16, 40) zurückgehaltenen Analyten der ersten Analyt-Klasse mittels einer Waschlösung, ein Eluieren der Analyten von der ersten Isolationsstruktur (16, 40) mittels einer Eluierungslösung und ein Transferieren der von der ersten Isolationsstruktur (16, 40) gelösten Analyten in eine erste Sammelkammer (32, 42, 87) aufweist.

3. Verfahren nach einem der Ansprüche 1 oder 2, das ein Mischen des Probenvolumens mit einem Bindepuffer, das in Kontaktbringen des dadurch erhaltenen Gemisches mit der Oberfläche, um die Analyten der zweiten Analyt-Klasse an der Oberfläche zu binden, ein Waschen der Oberfläche mit den gebundenen Analyten unter Verwendung eines Waschpuffers, ein Eluieren der Analyten von der Oberfläche unter Verwendung eines Elutionspuffers, und ein Transferieren der von der zweiten Isolationsstruktur (18, 44) gelösten Analyten in eine zweite Sammelkammer (34, 46, 90) aufweist.

4. Verfahren nach einem der Ansprüche 1 bis 3, das vor dem Leiten des Probenvolumens in die erste Isolationskammer (12, 85) ein Leiten des Probenvolumens durch ein vorgeschaltetes Filter (54) aufweist, das für die Analyten der ersten Analyt-Klasse und die Analyten der zweiten Analyt-Klasse durchlässig ist, um Zellen, Zellfragmente, Partikel und Verunreinigungen aus dem Probenvolumen zu filtern.

5. Verfahren nach einem der Ansprüche 1 bis 4, das ein Vorbereiten der biologischen Probe aufweist, wobei das Vorbereiten einen oder mehrere der folgenden Prozesse aufweist:
Durchführen einer Blut-Plasma-Separation der biologischen Probe, um Plasma zu erhalten, aus dem das Probenvolumen gewonnen wird;
Durchführen einer Blut-Plasma-Separation einer koagulierten biologischen Probe, um Serum zu erhalten, aus dem das Probenvolumen gewonnen wird;
Durchführen einer Thrombozyten-Isolation des Plasmas, um Thrombozyten-armes Plasma zu erhalten, aus dem das Probenvolumen gewonnen wird;
Durchführen einer Thrombozyten-Isolation des Plasmas, um Thrombozyten-reiches Plasma zu erhalten, aus dem das Probenvolumen gewonnen wird;
Durchführen eines enzymatischen Verdaus, um Proteine und Proteinaggregate in der biologischen Probe abzubauen, um das Probenvolumen zu gewinnen; und
Durchführen einer Verflüssigung und/oder Homogenisierung der biologischen Probe, um das Probenvolumen zu gewinnen.

6. Zentrifugal mikrofluidische Vorrichtung mit einem Fluidikmodul (10, 110, 132) zum Isolieren von Analyten einer ersten Analyt-Klasse, die extrazelluläre Vesikel und/oder zirkulierende Tumorzellen sind, und Analyten einer zweiten Analyt-Klasse, die zellfreie Nukleinsäuren sind, aus dem gleichen Probenvolumen einer biologischen Probe in einem zentrifugal-mikrofluidischen System, und einem Prozessierungsgerät, das ausgelegt ist, um das Fluidikmodul mit einer Rotation zu beaufschlagen,
wobei das Fluidikmodul folgende Merkmale aufweist:
eine erste Isolationskammer (12, 85), welche eine erste Isolationsstruktur (16, 40) enthält;
eine zweite Isolationskammer (14, 88), welche eine zweite Isolationsstruktur (18, 44) enthält;
eine erste Fluidleitung (22, 47) zum Leiten des Probenvolumens in die erste Isolationskammer (12, 85), so dass die Analyten der ersten Analyt-Klasse durch die erste Isolationsstruktur (16, 40) zurückgehalten werden, während die Analyten der zweiten Analyt-Klasse nicht von der ersten Isolationsstruktur (16, 40) zurückgehalten werden und als Teil einer Restflüssigkeit die erste Isolationsstruktur (16, 40) passieren;
eine zweite Fluidleitung (24, 48) zum Leiten der Restflüssigkeit in die zweite Isolationskammer (14, 88), so dass die Analyten der zweiten Analyt-Klasse durch die zweite Isolationsstruktur (18, 44) zurückgehalten werden;
eine erste Sammelkammer (32, 42, 87), die mit der ersten Isolationskammer (12, 85) verbunden ist, zur Aufnahme von von der ersten Isolationsstruktur (16, 40) gelösten Analyten der ersten Analyt-Klasse; und
eine zweite Sammelkammer (34, 46, 90), die mit der zweiten Isolationskammer (14, 88) verbunden ist, zur Aufnahme von von der zweiten Isolationsstruktur (18, 44) gelösten Analyten der zweiten Analyt-Klasse oder zur Aufnahme der zweiten Isolationsstruktur, von der die Analyten der zweiten Analyt-Klasse gelöst wurden,
wobei die erste Isolationsstruktur (16, 40) ein Filter mit einer Porengröße in einem Bereich von 20 Nanometern bis 200 Nanometern, vorzugsweise in einem Bereich von 20 Nanometern bis 45 Nanometern ist,
wobei die zweite Isolationsstruktur (18, 44) eine Oberfläche zum Binden von Analyten der zweiten Analyt-Klasse ist, und.
wobei die Oberfläche durch magnetisierbare Partikel gebildet ist,
wobei das Prozessierungsgerät einen oder mehrere stationäre oder verfahrbare Magneten aufweist, die außerhalb der zweiten Isolationskammer (14, 88) angeordnet sind, um bei einer Rotation des Fluidikmoduls (10, 110, 132) ein Durchmischen magnetisierbarer Partikel mit dem Filtrat zu unterstützen und/oder um bei einem Transfer von Analyten der zweiten Analyt-Klasse in die zweite Sammelkammer ein Zurückhalten der magnetisierbaren Partikel in der zweiten Isolationskammer (14, 88) zu unterstützen.

7. Zentrifugal mikrofluidische Vorrichtung nach Anspruch 6, bei der das Fluidikmodul (10, 110, 132) zumindest ein Filter (54) aufweist, das mit der Eingangsseite der ersten Isolationskammer (12, 85) fluidisch verbunden ist, das für die Analyten der ersten Analyt-Klasse und die Analyten der zweiten Analyt-Klasse durchlässig ist und das ausgelegt ist, um Zellen, Zellfragmente, Partikel und Verunreinigungen aus dem Probenvolumen zu filtern.

## Claims

1. A method for isolating analytes of a first analyte class, which are extracellular vesicles and/or circulating tumor cells, and analytes of a second analyte class, which are cell-free nucleic acids, from the same sample volume of a biological sample in a centrifugal microfluidic system, comprising a fluidics module (10, 110, 132) having a first isolation chamber (12, 85) containing a first isolation structure (16, 40) and a second isolation chamber (14, 88) containing a second isolation structure (18, 44), comprising:
guiding the sample volume into the first isolation chamber (12, 85) so that the analytes of the first analyte class are retained by the first isolation structure (16, 40), while the analytes of the second analyte class are not retained by the first isolation structure (16, 40) and pass through the first isolation structure (16, 40) as part of a residual liquid;
guiding the residual liquid into the second isolation chamber (14, 88) so that the analytes of the second analyte class are retained by the second isolation structure (18, 44); and
separating the analytes of the first analyte class from the first isolation structure (16, 40) and separating the analytes of the second analyte class from the second isolation structure (18, 44) to provide the analytes of the first analyte class and the analytes of the second analyte class separately from each other for subsequent analysis,
wherein the first isolation structure (16, 40) is a filter having a pore size in a range from 20 nanometers to 200 nanometers, preferably in a range from 20 nanometers to 45 nanometers,
wherein the second isolation structure (18, 44) is a surface for binding analytes of the second analyte class, wherein the surface is formed by particles, and
wherein the particles are magnetizable, the method comprising rotating the fluidics module (10, 110, 132) to, by means of one or more stationary or movable magnets (140) of a processing apparatus, move the magnetizable particles in the second isolation chamber (14, 88) to assist mixing of the particles with the residual liquid, and/or support retaining the magnetizable particles in the second isolation chamber (14, 88) during transfer of analytes of the second analyte class into the second collection chamber.

2. The method according to claim 1, comprising washing the analytes of the first analyte class retained at the first isolation structure (16, 40) by means of a washing solution, eluting the analytes from the first isolation structure (16, 40) by means of an elution solution and transferring the analytes detached from the first isolation structure (16, 40) into a first collection chamber (32, 42, 87).

3. The method according to any one of claims 1 or 2, comprising mixing the sample volume with a binding buffer, bringing the resulting mixture into contact with the surface to bind the analytes of the second analyte class to the surface, washing the surface with the bound analytes using a wash buffer, eluting the analytes from the surface using an elution buffer, and transferring the analytes detached from the second isolation structure (18, 44) to a second collection chamber (34, 46, 90).

4. The method according to any one of claims 1 to 3, comprising, prior to guiding the sample volume into the first isolation chamber (12, 85), guiding the sample volume through an upstream filter (54) which is permeable to the analytes of the first analyte class and the analytes of the second analyte class in order to filter cells, cell fragments, particles and impurities from the sample volume.

5. The method according to any one of claims 1 to 4, comprising preparing the biological sample, wherein preparing comprises one or more of the following processes:
performing blood-plasma separation of the biological sample to obtain plasma from which the sample volume is recovered;
performing blood-plasma separation of a coagulated biological sample to obtain serum from which the sample volume is recovered;
performing thrombocyte isolation of the plasma to obtain thrombocyte-poor plasma from which the sample volume is recovered;
performing thrombocyte isolation of the plasma to obtain thrombocyte-rich plasma from which the sample volume is recovered;
performing enzymatic digestion to decompose proteins and protein aggregates in the biological sample to recover the sample volume; and
performing liquefaction and/or homogenization of the biological sample to recover the sample volume.

6. A centrifugal microfluidic device comprising a fluidics module (10, 110, 132) for isolating analytes of a first analyte class, which are extracellular vesicles and/or circulating tumor cells, and analytes of a second analyte class, which are cell-free nucleic acids, from the same sample volume of a biological sample in a centrifugal microfluidic system, and a processing apparatus configured to subject the fluidics module to rotation,
the fluidics module comprising:
a first isolation chamber (12, 85) containing a first isolation structure (16, 40);
a second isolation chamber (14, 88) containing a second isolation structure (18, 44);
a first fluid line (22, 47) for guiding the sample volume into the first isolation chamber (12, 85) so that the analytes of the first analyte class are retained by the first isolation structure (16, 40), while the analytes of the second analyte class are not retained by the first isolation structure (16, 40) and pass through the first isolation structure (16, 40) as part of a residual liquid;
a second fluid line (24, 48) for guiding the residual liquid into the second isolation chamber (14, 88) so that the analytes of the second analyte class are retained by the second isolation structure (18, 44);
a first collection chamber (32, 42, 87) connected to the first isolation chamber (12, 85) for receiving analytes of the first analyte class detached from the first isolation structure (16, 40); and
a second collection chamber (34, 46, 90) connected to the second isolation chamber (14, 88) for receiving analytes of the second analyte class detached from the second isolation structure (18, 44) or for receiving the second isolation structure from which the analytes of the second analyte class have been detached,
wherein the first isolation structure (16, 40) is a filter having a pore size in a range from 20 nanometers to 200 nanometers, preferably in a range from 20 nanometers to 45 nanometers,
wherein the second isolation structure (18, 44) is a surface for binding analytes of the second analyte class, and
wherein the surface is formed by magnetizable particles,
wherein the processing apparatus comprises one or more stationary or movable magnets which are arranged outside the second isolation chamber (14, 88) in order to support mixing of magnetizable particles with the filtrate during rotation of the fluidics module (10, 110, 132) and/or in order to support retention of the magnetizable particles in the second isolation chamber (14, 88) during transfer of analytes of the second analyte class into the second collection chamber.

7. The centrifugal microfluidic device according to claim 6, wherein the fluidics module (10, 110, 132) comprises at least one filter (54) fluidically connected to the input side of the first isolation chamber (12, 85), which is permeable to the analytes of the first analyte class and the analytes of the second analyte class and which is configured to filter cells, cell fragments, particles and impurities from the sample volume.

## Revendications

1. Procédé destiné à isoler des analytes d'une première classe d'analytes, qui sont des vésicules extracellulaires et/ou des cellules tumorales circulantes, et des analytes d'une seconde classe d'analytes, qui sont des acides nucléiques acellulaires, à partir du même volume d'échantillon d'un échantillon biologique dans un système microfluidique centrifuge qui présente un module fluidique (10, 110, 132), lequel présente une première chambre d'isolement (12, 85) qui contient une première structure d'isolement (16, 40) ainsi qu'une seconde chambre d'isolement (14, 88) qui contient une seconde structure d'isolement (18, 44) avec les caractéristiques suivantes :
guider le volume d'échantillon dans la première chambre d'isolement (12, 85) de telle sorte que les analytes de la première classe d'analytes soient retenus par l'intermédiaire de la première structure d'isolement (16, 40) tandis que les analytes de la seconde classe d'analytes ne sont pas retenus par la première structure d'isolement (16, 40) et passent la première structure d'isolement (16, 40) en tant que partie d'un liquide résiduel ;
guider le liquide résiduel dans la seconde chambre d'isolement (14, 88), de telle sorte que les analytes de la seconde classe d'analytes soient retenus par l'intermédiaire de la seconde structure d'isolement (18, 44) ; et
séparer les analytes de la première classe d'analytes de la première structure d'isolement (16, 40) et séparer les analytes de la seconde classe d'analytes de la seconde structure d'isolement (18, 44) afin de fournir séparément les uns des autres les analytes de la première classe d'analytes et les analytes de la seconde classe d'analytes pour une analyse ultérieure,
dans lequel la première structure d'isolement (16, 40) est un filtre avec une taille de pores comprise dans une plage de 20 nanomètres à 200 nanomètres, de préférence dans une plage de 20 nanomètres à 45 nanomètres,
dans lequel la seconde structure d'isolement (18, 44) est une surface destinée à lier des analytes de la seconde classe d'analytes, dans lequel la surface est formée par l'intermédiaire de particules,
dans lequel les particules sont magnétisables, dans lequel le procédé présente une rotation du module fluidique (10, 110, 132) afin de déplacer les particules magnétisables dans la seconde chambre d'isolement (14, 88) à l'aide d'un ou plusieurs aimants fixes ou mobiles (140) d'un appareil de traitement pour promouvoir un mélange des particules avec le liquide résiduel et/ou promouvoir une retenue des particules magnétisables dans la seconde chambre d'isolement (14, 88) lors d'un transfert d'analytes de la seconde classe d'analytes dans la seconde chambre de collecte.

2. Procédé selon la revendication 1, lequel présente un lavage des analytes de la première classe d'analytes retenus sur la première structure d'isolement (16, 40) à l'aide d'une solution de lavage, une élution des analytes de la première structure d'isolement (16, 40) à l'aide d'une solution d'élution et un transfert des analytes dissous de la première structure d'isolement (16, 40) dans une première chambre de collecte (32, 42, 87).

3. Procédé selon une des revendications 1 ou 2, qui présente un mélange du volume d'échantillon avec un tampon de liaison, la mise en contact du mélange ainsi obtenu avec la surface pour lier les analytes de la seconde classe d'analytes sur la surface, un lavage de la surface avec les analytes liés en utilisant un tampon de lavage, une élution des analytes de de la surface en utilisant un tampon d'élution, et un transfert des analytes dissous de la seconde structure d'isolement (18, 44) dans une seconde chambre de collecte (34, 46, 90).

4. Procédé selon une des revendications 1 à 3 qui présente, avant de guider le volume d'échantillon dans la première chambre d'isolement (12, 85), un guidage du volume d'échantillon par l'intermédiaire d'un filtre disposé en amont (54) qui est perméable aux analytes de la première classe d'analytes et aux analytes de la seconde classe d'analytes afin de filtrer les cellules, les fragments de cellules, les particules et les contaminants du volume d'échantillon.

5. Procédé selon une des revendications 1 à 4, qui présente une préparation de l'échantillon biologique, dans lequel la préparation présente un ou plusieurs des processus suivants :
réaliser une séparation sang-plasma de l'échantillon biologique afin de recueillir le plasma à partir duquel le volume d'échantillon est obtenu ;
réaliser une séparation sang-plasma d'un échantillon biologique coagulé afin de recueillir le sérum à partir duquel le volume d'échantillon est obtenu ;
réaliser un isolement des thrombocytes du plasma afin de recueillir un plasma pauvre en thrombocytes à partir duquel le volume d'échantillon est obtenu ;
réaliser un isolement des thrombocytes du plasma afin de recueillir un plasma riche en thrombocytes à partir duquel le volume d'échantillon est obtenu ;
réaliser une digestion enzymatique afin de diminuer les protéines et les agrégats de protéines dans l'échantillon biologique pour obtenir le volume d'échantillon, et
réaliser une liquéfaction et/ou homogénéisation de l'échantillon biologique pour obtenir le volume d'échantillon.

6. Dispositif microfluidique centrifuge avec un module fluidique (10, 110, 132) destiné à isoler des analytes d'une première classe d'analytes circulantes, qui sont des vésicules extracellulaires et/ou des cellules tumorales, et les analytes d'une seconde classe d'analytes, qui sont des acides nucléiques acellulaires, à partir du même volume d'échantillon d'un échantillon biologique dans un système microfluidique centrifuge, et un appareil de traitement conçu pour solliciter en rotation le module fluidique,
dans lequel le module fluidique présente les caractéristiques suivantes :
une première chambre d'isolement (12, 85) qui contient une première structure d'isolement (16, 40) ;
une seconde chambre d'isolement (14, 88) qui contient une seconde structure d'isolement (18, 44) ;
une première conduite de fluide (22, 47) pour guider le volume d'échantillon dans la première chambre d'isolement (12, 85), de telle sorte que les analytes de la première classe d'analytes soient retenus par l'intermédiaire de la première structure d'isolement (16, 40), tandis que les analytes de la seconde classe d'analytes ne sont pas retenus par la première structure d'isolement (16, 40) et passent la première structure d'isolement (16, 40) en tant que partie d'un liquide résiduel ;
une seconde conduite de fluide (24, 48) pour guider le liquide résiduel dans la seconde chambre d'isolement (14, 88), de telle sorte que les analytes de la seconde classe d'analytes soient retenus par l'intermédiaire de la seconde structure d'isolement (18, 44) ;
une première chambre de collecte (32, 42, 87) qui est reliée à la première chambre d'isolement (12, 85) pour recevoir les analytes de la première classe d'analytes dissouts par la première structure d'isolement (16, 40) ; et
une seconde chambre de collecte (34, 46, 90) qui est reliée à la seconde chambre d'isolement (14, 88) pour recevoir les analytes de la seconde classe d'analytes dissouts par la seconde structure d'isolement (18, 44) ou pour recevoir la seconde structure d'isolement, à partir de laquelle les analytes de la seconde classe d'analytes ont été dissouts,
dans lequel la première structure d'isolement (16, 40) est un filtre avec une taille de pores comprise dans une plage de 20 nanomètres à 200 nanomètres, de préférence dans une plage de 20 nanomètres à 45 nanomètres,
dans lequel la seconde structure d'isolement (18, 44) est une surface destinée à lier des analytes de la seconde classe d'analytes, et
dans lequel la surface est formée par l'intermédiaire de particules magnétisables,
dans lequel l'appareil de traitement présente un ou plusieurs aimants fixes ou mobiles qui sont disposés en dehors de la seconde chambre d'isolement (14, 88) pour promouvoir un mélange de particules magnétisables avec le filtrat lors d'une rotation du module fluidique (10, 110, 132) et/ou pour promouvoir une retenue des particules magnétisables dans la seconde chambre d'isolement (14, 88) lors d'un transfert d'analytes de la seconde classe d'analytes dans la seconde chambre de collecte.

7. Dispositif microfluidique centrifuge selon la revendication 6, dans lequel le module fluidique (10, 110, 132) présente au moins un filtre (54) qui est relié de manière fluidique au côté d'entrée de la première chambre d'isolement (12, 85), qui est perméable aux analytes de la première classe d'analytes et aux analytes de la seconde classe d'analytes et qui est conçu pour filtrer des cellules, des fragments de cellules, des particules et des contaminants du volume d'échantillon.
